# EUROPEAN PATENT APPLICATION

(11) **EP 4 640 668 A1**
(43) Date of publication of application: **29.10.2025**
(21) Application number: 24197724.8
(22) Date of filing: 30.08.2024
(51) Int. Cl.: C07C 233/36, C07C 235/10

(54) **LIPID COMPOUND, LIPID NANOPARTICLE COMPRISING SAME AND USE THEREOF**

(30) Priority: 23.04.2024 CN 202410487810
(71) Applicant: TheMedium Therapeutics Co., Ltd., Suzhou, Jiangsu (CN)
(72) Inventor: ZHANG, Lei, Suzhou (CN); CHEN, Jianxin, Suzhou (CN)
(74) Representative: V.O.

(57) **Abstract**

The present disclosure provides a lipid compound, a lipid nanoparticle comprising the same and use thereof in the preparation of a drug delivery carrier. The lipid nanoparticle of the present disclosure can deliver a therapeutic or prophylactic agent (particularly a nucleic acid substance) to an injection site by intramuscular injection, and the therapeutic or prophylactic agent has high expression at the injection site but low expression in the viscera, which enables a drug to not only exert high activity but also have low visceral toxicity, and is of great significance to development and application of nucleic acid therapeutic or prophylactic agents.

## Description

### CROSS-REFERENCE TO RELATED APPLICATION

This application claims priority to Chinese Patent Application No. 202410487810.8 filed April 23, 2024, the disclosure of which is incorporated herein by reference in its entirety.

### TECHNICAL FIELD

The present disclosure belongs to the field of molecular biology, and particularly relates to a lipid compound, a lipid nanoparticle comprising the same and use thereof in the preparation of a drug delivery carrier, and a pharmaceutical composition and an agent based on the lipid nanoparticle.

### BACKGROUND

Therapeutic or prophylactic nucleic acids have the potential to revolutionize vaccination, gene therapy, protein alternative therapy, and other therapies for genetic diseases. Since the first clinical study on therapeutic nucleic acids in 2000, significant advances have been made in the research on the design of nucleic acid molecules and the method of delivering them. However, nucleic acid drugs, including therapeutic and prophylactic drugs, still face several challenges, such as the accumulation of most liposome formulations through biological processes in the liver, thereby reducing the efficacy of compositions delivered into target sites. Therefore, a lipid nanoparticle delivery system with reduced liver enrichment, high specificity, and high delivery capacity still needs to be developed.

### SUMMARY

### Purpose of the Present Disclosure

The present disclosure aims to provide a cationic lipid compound capable of delivering a nucleic acid therapeutic or prophylactic agent, a lipid nanoparticle comprising the same and use thereof in the preparation of a drug delivery carrier, and a pharmaceutical composition and an agent based on the lipid nanoparticle.

The drug delivery carrier based on the cationic lipid compound of the present disclosure can effectively deliver a nucleic acid therapeutic or prophylactic agent to an injection site by intramuscular injection, and can achieve high expression of nucleic acid at the injection site but low expression in the viscera, thereby being capable of reducing toxicity.

### Solution

In order to achieve the purpose of the present disclosure, the following technical solutions are provided:

In a first aspect, the present invention provides a compound represented by formula (I) or a pharmaceutically acceptable salt, a prodrug, or a stereoisomer thereof, 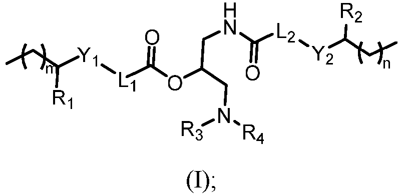 wherein:
Y₁ and Y₂ are each independently -OC (=O)- or -C (=O)O-;
L₁ and L₂ are each independently a bond or optionally substituted C₁-C₁₈ alkylene;
R₁ and R₂ are each independently H or C₁-C₈ linear or branched alkyl;
R₃ and R₄ are each independently C₁-C₈ linear or branched alkyl optionally substituted with hydroxyl;
m is an integer of 1-10;
n is an integer of 1-10.

Preferably, L₁ and L₂ are each independently unsubstituted C₂-C₁₄ alkylene.

Preferably, R₁ and R₂ are each independently H or C₁-C₈ linear alkyl; more preferably, R₁ and R₂ are each independently H or C₆-C₈ linear alkyl.

Preferably, R₃ and R₄ are each independently C₁-C₄ linear or branched alkyl optionally substituted with hydroxyl; more preferably, R₃ and R₄ are each independently methyl, ethyl, propyl, hydroxymethyl, hydroxyethyl, hydroxypropyl, or hydroxybutyl; even more preferably, R₃ and R₄ are each independently methyl or hydroxyethyl.

Preferably, m is an integer of 5-7;
and preferably, n is an integer of 5-7.

In a preferred embodiment, the compound is selected from the following compounds:

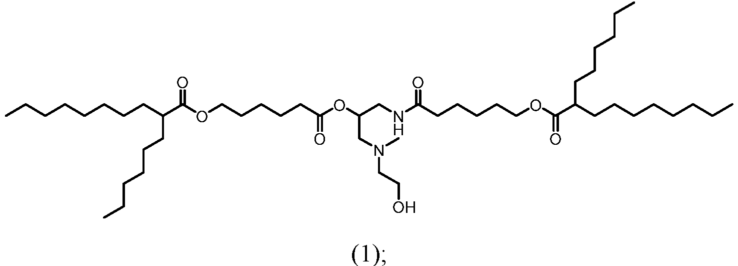

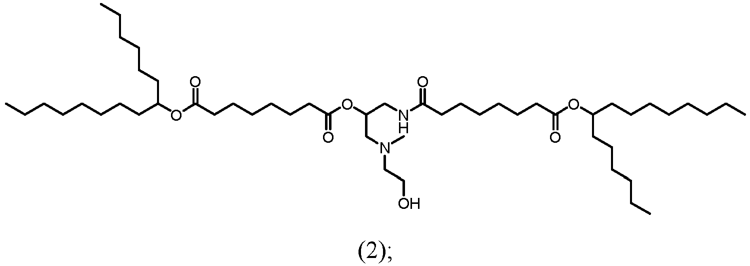

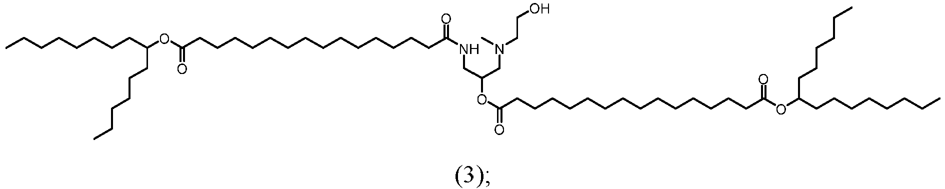

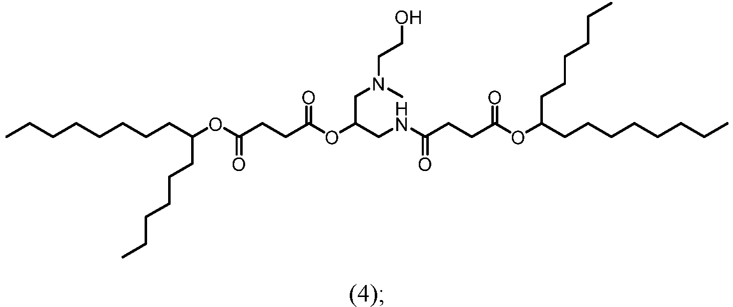

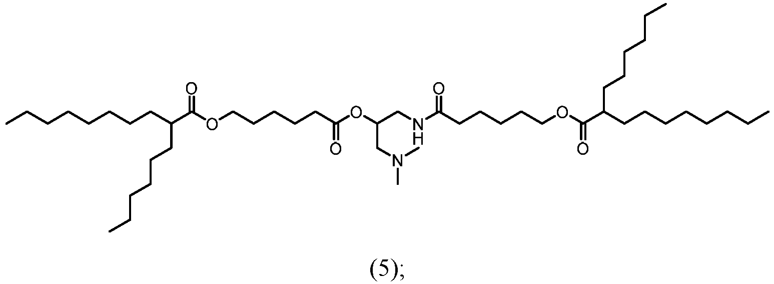

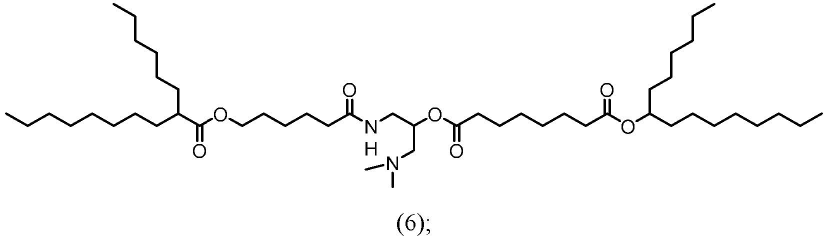

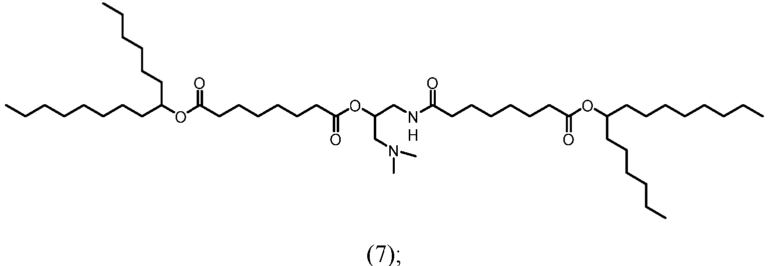

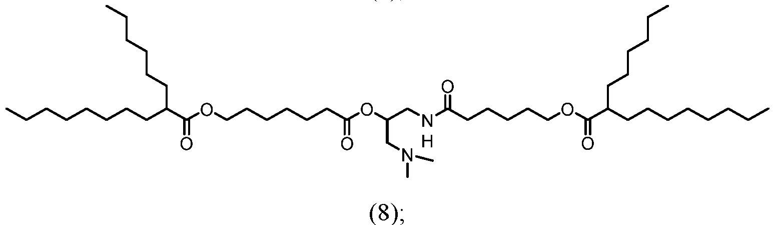

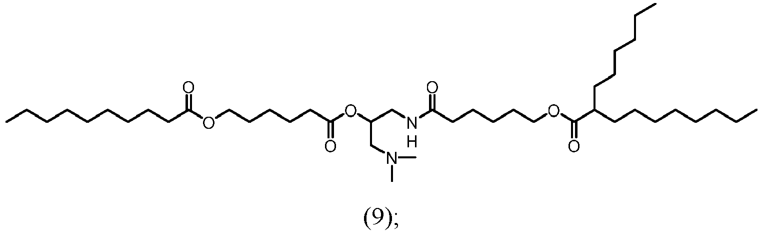

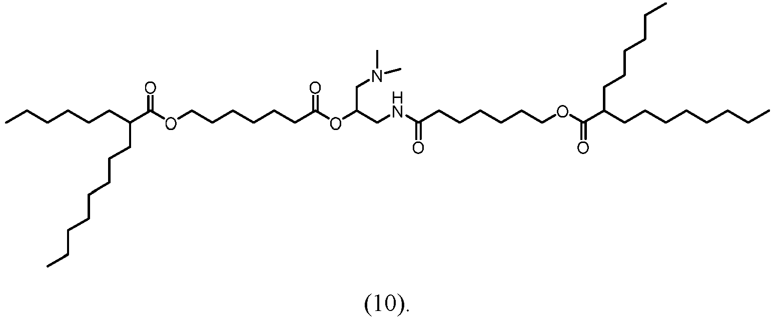

Preferably, the compound is selected from compound 1, 2, 3, 4, 5, 6, 7, or 10 as shown in the above table, more preferably compound 3, 4, 5, or 7, even more preferably compound 3, 5, or 7, most preferably compound 5 or 7.

In the above aspect, the "pharmaceutically acceptable salt" refers to a salt of a compound of the present disclsoure that is pharmaceutically acceptable and has a desired pharmacological activity. Such salts include acid addition salts formed with the following acids: inorganic acids such as hydrochloric acid, hydrobromic acid, sulfuric acid, nitric acid, phosphoric acid, and the like; or organic acids such as 1,2-ethanedisulfonic acid, 2-hydroxyethanesulfonic acid, 2-naphthalenesulfonic acid, 3-phenylpropionic acid, 4,4'-methylene-bis(3-hydroxy-2-ene-1-methanoic acid), 4-methylbicyclo[222]oct-2-ene-1-methanoic acid, acetic acid, aliphatic monocarboxylic and dicarboxylic acids, aliphatic sulfuric acid, aromatic sulfuric acid, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, lauryl sulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acid, propionic acid, *p*-toluenesulfonic acid, pyruvic acid, salicylic acid, stearic acid, succinic acid, tartaric acid, *tert*-butylacetic acid, trimethylacetic acid, and the like. The pharmaceutically acceptable salts also include base addition salts which is formed when an acidic proton present is capable of reacting with an inorganic or organic base. The acceptable inorganic bases include, but are not limited to, sodium hydroxide, sodium carbonate, potassium hydroxide, aluminum hydroxide, and calcium hydroxide. The acceptable organic bases include, but are not limited to, ethanolamine, diethanolamine, triethanolamine, tromethamine, N-methylglucamine, and the like. It should be recognized that the particular anion or cation forming part of any salt of the present disclosure is not critical so long as the salt as a whole is pharmacologically acceptable.

In the above aspect, the "prodrug" refers to a compound, such as a therapeutic agent that can be converted into a biologically active compound under physiological conditions or by dissolution. The prodrug is generally rapidly converted *in vivo* to yield a parent compound, for example, by hydrolysis in blood. The prodrug compound generally has the advantage of solubility, histocompatibility, or delayed release in mammalian organisms. The term "prodrug" is also meant to include any covalently bonded carriers that release active compounds *in vivo* when the prodrug is administered to a mammalian subject. The prodrug includes a compound where hydroxyl, amino or sulfhydryl is bonded to any group that, when the prodrug is administered to the mammalian subject, cleaves to form free hydroxyl, free amino, or free sulfhydryl, respectively. Examples of the prodrugs include, but are not limited to, acetate, formate and benzoate derivatives or amide derivatives of amine functional groups and the like.

In the above aspect, the "stereoisomers" are isomers of such a given compound, wherein the same atom is bonded to the same other atom; however, the three-dimensional configuration of those atoms is different. "Enantiomers" are stereoisomers of a given compound that are mirror images of each other as left and right hands. "Diastereoisomers" are stereoisomers of a given compound that are not enantiomers. Chiral molecules contain a chiral center (also referred to as a stereocenter or a stereogenic center), which is any point (although not necessarily an atom) in the molecule that carries multiple groups, such that the interchange of any 2 groups produces a stereoisomer. In organic compounds, the chiral center is typically a carbon, phosphorus, or sulfur atom, although other atoms may also be stereocenters in organic and inorganic compounds. A molecule can have multiple stereocenters, giving rise to many of its stereoisomers. In compounds of which stereoisomerism is due to tetrahedral stereogenic centers (e.g., tetrahedral carbon), it is assumed that the total number of possible stereoisomers does not exceed 2n, wherein n is the number of tetrahedral stereogenic centers. Molecules with symmetry often have a number that is smaller than the maximum possible number of stereoisomers. A 50:50 mixture of enantiomers is referred to as a racemic mixture. Alternatively, a mixture of enantiomers can be enriched such that one enantiomer is present in an amount greater than 50%. Generally, enantiomers and/or diastereoisomers can be resolved or separated using techniques known in the art. It is contemplated that for any stereogenic center or chiral axis for which stereochemistry has not been defined, the stereogenic center or chiral axis can exist in its R form, S form, or as a mixture of the R form and the S form (including racemic and non-racemic mixtures). As used herein, the phrase "substantially free of other stereoisomers" means that the composition contains 15% or less, more preferably 10% or less, even more preferably 5% or less, or most preferably 1% or less of another or more stereoisomers.

In a second aspect, the present invention provides a lipid nanoparticle comprising the compound or the pharmaceutically acceptable salt, the prodrug, or the stereoisomer thereof according to the first aspect described above.

Further, the lipid nanoparticle can further comprise at least one helper lipid, which is mixed with a drug or a molecule/agent with pharmaceutical activity to achieve encapsulation and achieve effective delivery of the drug.

Optionally, the helper lipid is one or more of a phospholipid, a steroid, a polymer conjugated lipid, and a modifiable lipid.

Preferably, the phospholipid is any one or a combination of two or more of DOPE, DSPC, DPPC, DMPC, DOPC, POPC, and SM, more preferably DOPE or DSPC. In a preferred embodiment, the phospholipid is DOPE. In another preferred embodiment, the phospholipid is DSPC.

Preferably, the steroid is any one or a combination of two or more of cholesterol, sitosterol, stigmasterol, and ergosterol; more preferably, the steroid is cholesterol and/or sitosterol.

With regard to the polymer conjugated lipid described above, the polymer is a high-molecular-weight compound formed by covalent bonding of one or more small-molecule repeating units; the polymer can be selected from polyethylene glycol, polylactic acid, polyamide, cationic polymer, poly-sarcosine (pSar), poly(lactic-co-glycolic acid) (PLGA), polyamino acid, polypeptide, polypeptoid, etc.; preferably, the polymer conjugated lipid is a polyethylene glycol conjugated lipid; more preferably, the polyethylene glycol conjugated lipid is selected from one or more of ALC-0159, PEG1000-DMG, PEG5000-DMG, PEG2000-DMG, and PEG2000-DSPE.

Preferably, the modifiable lipid includes lipids that can be modified by small-molecule compounds, vitamins, carbohydrates, peptides, proteins, nucleic acids, lipopolysaccharides, inorganic molecules or particles, metal ions or particles, and combinations of the substances described above.

For the lipid nanoparticle described above, preferably, the compound or the pharmaceutically acceptable salt, the prodrug, or the stereoisomer thereof according to the first aspect described above and the helper lipid are in a molar ratio of 1:(0.5-2), preferably 1:(0.6-1.5), and more preferably 1:(0.8-1.2).

The lipid nanoparticle can deliver therapeutic/prophylactic agents to the injection site by intramuscular injection.

In a third aspect, the present invention provides use of the compound or the pharmaceutically acceptable salt, the prodrug, or the stereoisomer thereof according to the first aspect described above, or the lipid nanoparticle according to the second aspect described above in the preparation of a drug delivery carrier.

Viably, the drug is a therapeutic or prophylactic agent.

Further viably, the therapeutic or prophylactic agent is a nucleic acid.

The nucleic acid includes any and all forms of nucleic acid molecules, including other forms of nucleic acid molecules known in the art or likely to be discovered/prepared in the future; in a preferred embodiment, the nucleic acid is selected from a single-stranded DNA, a double-stranded DNA, a single-stranded RNA, a double-stranded RNA, a short isomer, a plasmid DNA, a complementary DNA (cDNA), an antisense oligonucleotide (ASO), a small interfering nucleic acid (siRNA), a small activating nucleic acid (saRNA), an asymmetric interfering nucleic acid (aiRNA), a micro nucleic acid (miRNA), a miRNA agonist (agomir), a miRNA antagonist (antagomir), a Dicer enzyme substrate nucleic acid, a small hairpin nucleic acid (shRNA), a transfer RNA (tRNA), a messenger RNA (mRNA), a circular RNA (circRNA), a self-amplifying mRNA (samRNA), and an aptamer.

The nucleic acid molecule can be a natural nucleotide, a nucleotide mimic, a functional analog, or a chemically modified nucleotide.

Functional nucleotide analogs include, but are not limited to, one of or a combination of more than one of a locked nucleic acid (LNA), a peptide nucleic acid (PNA), and a morpholine ring oligonucleotide nucleic acid mimic or functional analog.

The chemical modification of nucleotides can be located on a backbone bond of the nucleic acid molecule. The backbone bond can be modified by replacement of one or more oxygen atoms. The modification to the backbone bond can include replacing at least one phosphodiester bond with a phosphorothioate bond.

The chemical modification of nucleotides can also be located on a nucleoside. The modification on the nucleoside can be located on the sugar and base of the nucleoside. The modification of the sugar on the nucleoside can be selected from one or more of 2'-fluororibose, ribose, 2'-deoxyribose, arabinose, and hexose.

The chemically modified forms of nucleotides can be selected from one or more of: 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl) adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methylguanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. These modifications can be random or at specific sites.

In a preferred embodiment, the nucleic acid is mRNA.

Viably, the mRNA encodes at least one antigen or a fragment thereof or an epitope thereof, or encodes a certain therapeutic protein.

Further viably, the antigen is a pathogenic antigen such as a tumor-associated antigen or a pathogenic microbial antigen.

In addition, the mRNA can be a monocistronic mRNA or a polycistronic mRNA.

In a fourth aspect, the present disclosure provides a pharmaceutical composition comprising the lipid nanoparticle according to the second aspect described above and a therapeutic or prophylactic agent.

Preferably, the therapeutic or prophylactic agent is a nucleic acid, and the nucleic acid is encapsulated in the lipid nanoparticle.

Optional types of the nucleic acid are as defined in the third aspect described above.

In addition, preferably, in the pharmaceutical composition, the lipid nanoparticle and the therapeutic or prophylactic agent are in a mass ratio of 10:1 to 100:1, more preferably 20:1 to 50:1, and even more preferably 20: 1 to 30:1.

Preferably, the pharmaceutical composition has an average particle size of 90 nm to 600 nm, more preferably 200 nm to 400 nm, and even more preferably 200 nm to 300 nm.

Preferably, the pharmaceutical composition has a polydispersity index of 0.001 to 0.5, more preferably 0.001 to 0.45, and even more preferably 0.001 to 0.4.

In a fifth aspect, the present disclosure provides use of the pharmaceutical composition according to the fourth aspect described above in the preparation of a targeted delivery drug.

Viably, the drug comprises the composition described above and a pharmaceutically acceptable auxiliary material.

In a sixth aspect, the present invention provides a formulation comprising the pharmaceutical composition according to the fourth aspect described above and a pharmaceutically acceptable auxiliary material.

The "pharmaceutically acceptable auxiliary materials" described above refer to excipients and additives used in drug production and prescription dispensing and are substances other than the active ingredient, which have been rationally evaluated in safety and are contained in a pharmaceutical formulation. In addition to shaping, serving as vehicles, and enhancing stability, "pharmaceutically acceptable auxiliary materials" also have such important functions as solubilization, aiding dissolution, sustaining or controlling release, etc. They are important ingredients which possibly affect the quality, safety and effectiveness of drugs. According to the effect and the purpose, pharmaceutical auxiliary materials may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesives, integrating agents, permeation promotors, pH regulators, buffers, plasticizers, surfactants, foaming agents, defoaming agents, thickeners, inclusion agents, humectants, absorbents, diluents, flocculants, deflocculants, filter aids, release retardants, etc.

### Beneficial Effects

The present disclosure provides a cationic lipid compound, a lipid nanoparticle comprising the same and use thereof as a drug delivery carrier, and a pharmaceutical composition and an agent based on the lipid nanoparticle. The lipid nanoparticle of the present invention can deliver a therapeutic or prophylactic agent (particularly a nucleic acid substance) to an injection site by intramuscular injection, and the therapeutic or prophylactic agent has high expression at the injection site but low expression in the viscera, which enables a drug to not only exert high activity but also have low visceral toxicity, and is of great significance to development and application of nucleic acid therapeutic or prophylactic agents.

### BRIEF DESCRIPTION OF THE DRAWINGS

One or more embodiments are exemplarily illustrated by the figures in the corresponding drawings, which are not intended to limit the embodiments. The word "exemplary" is used exclusively herein to mean "serving as an example, embodiment, or illustration". Any embodiment described herein as "exemplary" is not necessarily to be construed as preferred or advantageous over other embodiments.

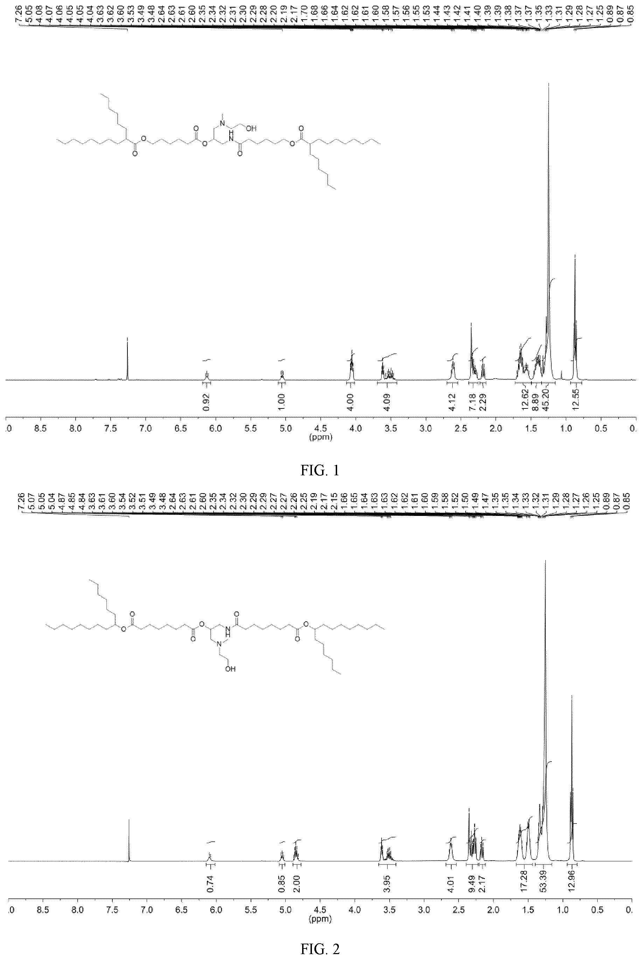

FIG. 1 is ¹H-NMR of compound 1.
FIG. 2 is ¹H-NMR of compound 2.

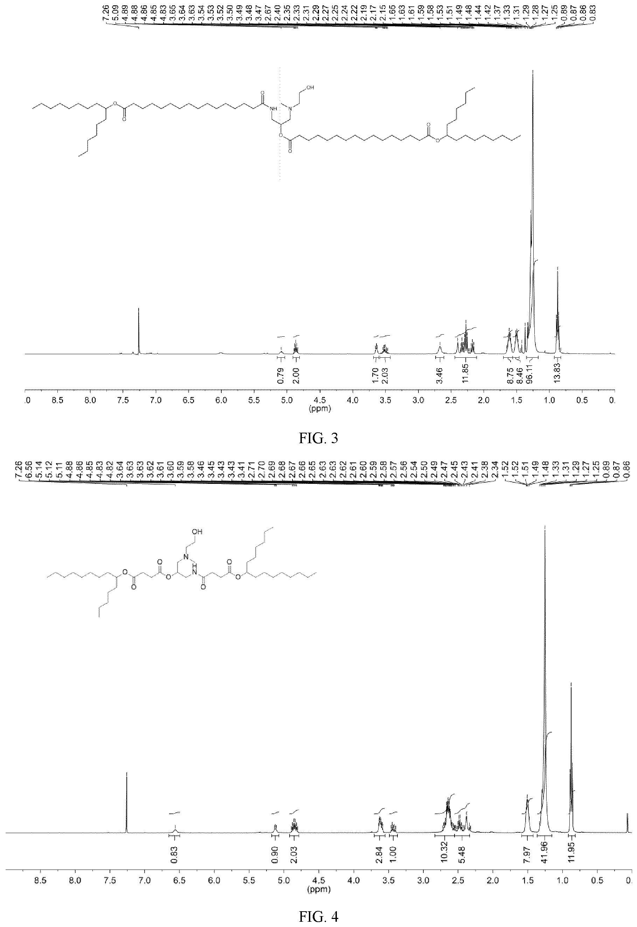

FIG. 3 is ¹H-NMR of compound 3.
FIG. 4 is ¹H-NMR of compound 4.

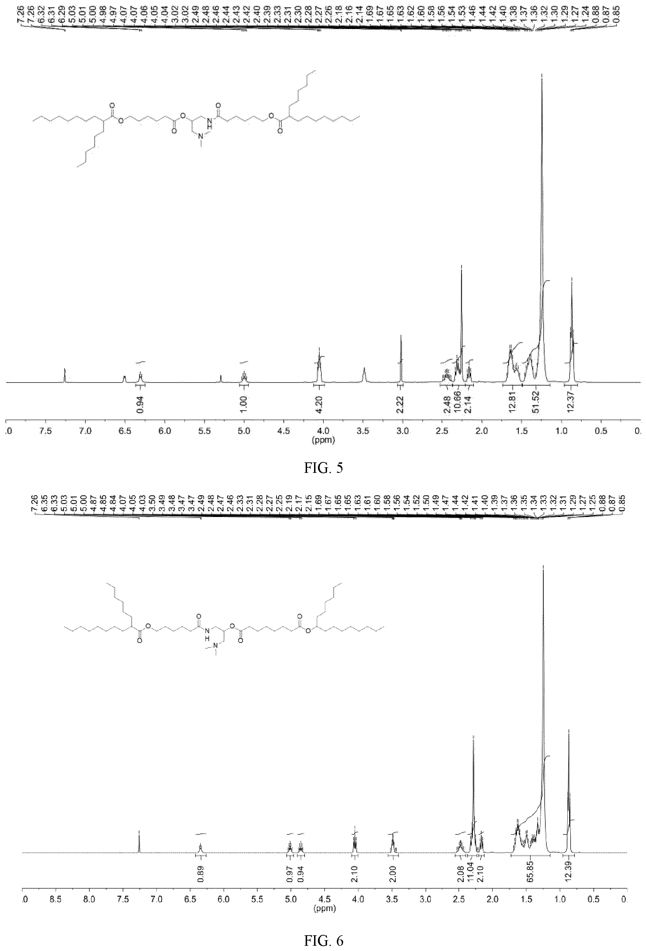

FIG. 5 is ¹H-NMR of compound 5.
FIG. 6 is ¹H-NMR of compound 6.

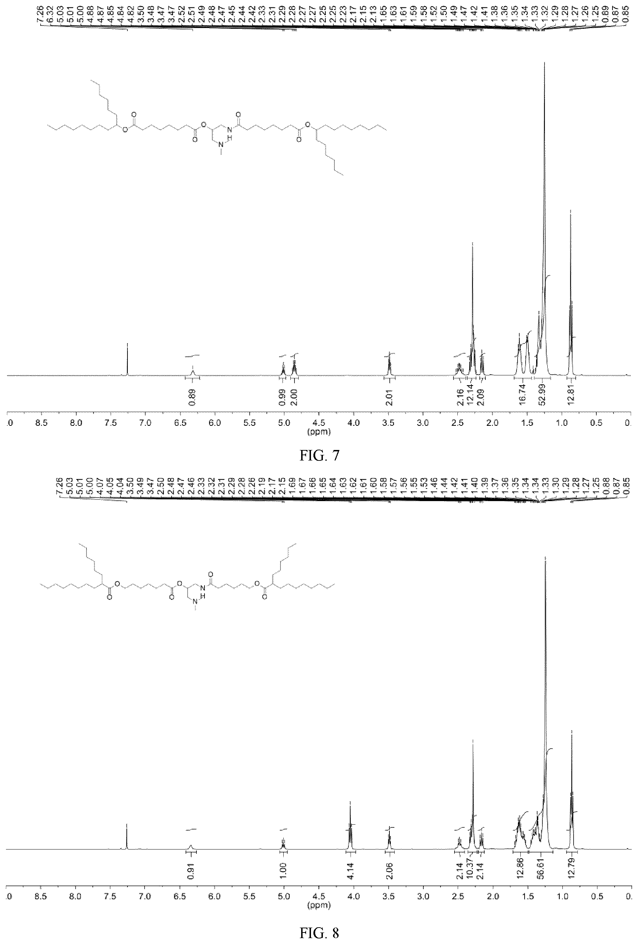

FIG. 7 is ¹H-NMR of compound 7.
FIG. 8 is ¹H-NMR of compound 8.

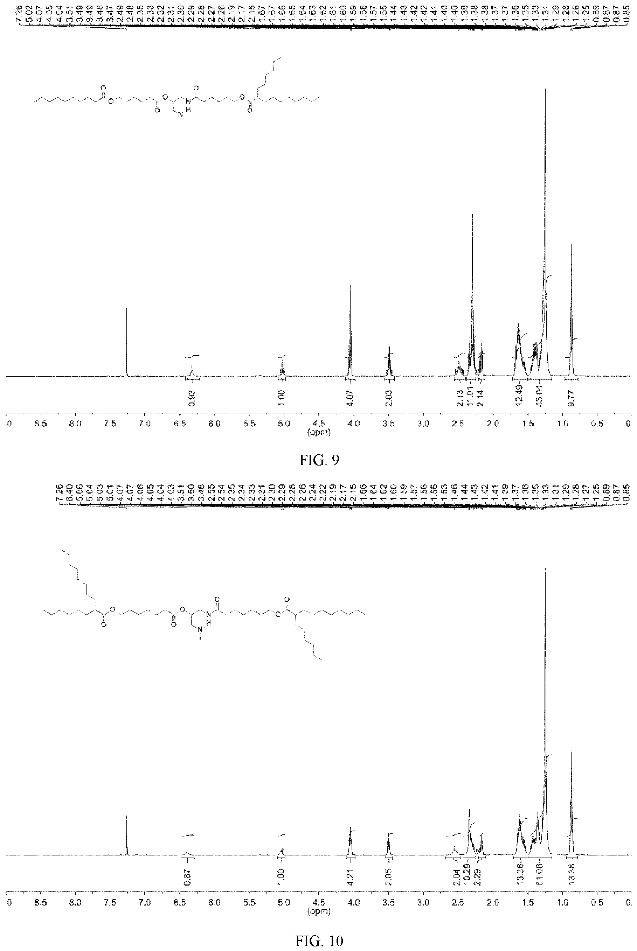

FIG. 9 is ¹H-NMR of compound 9.
FIG. 10 is ¹H-NMR of compound 10.
FIG. 11 shows summary data of fluorescence intensity ratios at the injection site and viscera after delivery by intramuscular injection of different lipid nanoparticles loaded with Luciferase mRNA.
FIG. 12 is a graph showing fluorescence signals of mice *in vivo* 4 h after administration by intramuscular injection of the lipid nanoparticle TMF 1 loaded with Luciferase mRNA; Color Scale: color scale; Min: minimum value; Max: maximum value.

### DETAILED DESCRIPTION

### A. Chemical and Formulation Definitions

When used in the context of a chemical group, "hydrogen" refers to -H; "deuterium" refers to ²H or D; "hydroxyl" refers to -OH; "oxo" refers to =O; "carbonyl" refers to -C(=O)-; "carboxyl" refers to -C(=O)OH (also written as -COOH or -CO₂H); "halo" independently refers to -F, -Cl, -Br, or -I; "amino" refers to -NH₂; "hydroxyamino" refers to -NHOH; "nitro" refers to -NO₂; imino refers to =NH; "cyano" refers to -CN; "isocyanate" refers to -N=C=O; "azido" refers to -N₃; in the context of a monovalent group, "phosphate" refers to -OP(O)(OH)₂ or a deprotonated form thereof; in the context of a divalent group, "phosphate" refers to -OP(O)(OH)O- or a deprotonated form thereof; "sulfhydryl" refers to -SH; and "thio" refers to =S; "sulfonyl" refers to -S(O)₂-; "hydroxysulfonyl" refers to -S(O)₂OH; "sulfonamide" refers to -S(O)₂NH₂; and "sulfinyl" refers to -S(O)-. In the context of a chemical formula, the symbol "-" refers to a single bond, "=" refers to a double bond, and "≡" refers to a triple bond. The symbol "----" represents an optional bond, which is a single or double bond, if present. When drawn perpendicularly across a bond, the symbol " - " indicates the point of attachment of the group. It should be noted that the point of attachment is generally identified only for larger groups in this manner to aid the reader in unequivocally identifying the point of attachment. The symbol " 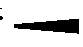 " refers to a single bond, wherein the group attached to the butt end of the wedge "emerges from the plane of the paper". The symbol " 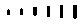 " refers to a single bond, wherein the group attached to the butt end of the wedge "goes into the plane of the paper". The symbol " - " refers to a single bond, wherein the geometry (e.g., E or Z) around the double bond is undefined. Thus, both options and combinations thereof are contemplated. Any undefined valence on an atom of a structure shown in the present application implicitly represents a hydrogen atom bonded to the atom. Bold dots on a carbon atom indicate that the hydrogen attached to the carbon is oriented out of the plane of the paper.

The term "alkyl" as used without the modifier "substituted" represents a monovalent saturated aliphatic group having a carbon atom as the point of attachment, having a linear or branched acyclic structure, and no atoms other than carbon and hydrogen. The groups -CH₃(Me), -CH₂CH₃(Et), -CH₂CH₂CH₃(n-Pr or propyl), -CH(CH₃)₂(i-Pr, iPr or isopropyl), - CH₂CH₂CH₂CH₃(n-Bu), -CH(CH₃)CH₂CH₃(sec-butyl), -CH₂CH(CH₃)₂(isobutyl), -C(CH₃)₃ (tert-butyl, t-Bu or tBu) and -CH₂C(CH₃)₃(neopentyl) are non-limiting examples of alkyl. The term "dialkyl" as used without the modifier "substituted" represents a divalent saturated aliphatic group having 1 or 2 saturated carbon atoms as the point of attachment, having a linear or branched acyclic structure, no carbon-carbon double or triple bonds, and no atoms other than carbon and hydrogen. The groups -CH₂-(methylene), -CH₂CH₂-, -CH₂C(CH₃)₂CH₂-, and -CH₂CH₂CH₂- are non-limiting examples of dialkyl. "Alkane" represents the class of compounds having formula H-R, wherein R is alkyl, which is as defined above. When any of these terms is used together with the modifier "substituted", one or more hydrogen atoms have been independently replaced by -OH, -F, -Cl, -Br, -I, -NH₂, -NO₂, -CO₂H, -CO₂CH₃, -CN, -SH, -OCH₃, -OCH₂CH₃, - C(O)CH₃, -NHCH₃, -NHCH₂CH₃, -N(CH₃)₂, -C(O)NH₂, -C(O)NHCH₃, -C(O)N(CH₃)₂, - OC(O)CH₃, -NHC(O)CH₃, -S(O)₂OH, or -S(O)₂NH₂. The following groups are non-limiting examples of substituted alkyl: -CH₂OH, -CH₂Cl, -CF₃, -CH₂CN, -CH₂C(O)OH, -CH₂C(O)OCH₃, -CH₂C(O)NH₂, -CH₂C(O)CH₃, -CH2OCH₃, -CH₂OC(O)CH₃, -CH₂NH₂, -CH₂N(CH₃)₂, and - CH₂CH₂Cl. The term "haloalkyl" is a subset of substituted alkyl, wherein hydrogen atom substitution is limited to halo (i.e., -F, -Cl, -Br, or -I), such that no atoms other than carbon, hydrogen and halogen are present. The group -CH₂Cl is one non-limiting example of haloalkyl. The term "fluoroalkyl" is a subset of substituted alkyl, wherein hydrogen atom substitution is limited to fluoro, such that no atoms other than carbon, hydrogen and fluorine are present. The groups -CH₂F, -CF₃, and -CH₂CF₃ are non-limiting examples of fluoroalkyl.

The term "alkenyl" as used without the modifier "substituted" represents a monovalent unsaturated aliphatic group having a carbon atom as the point of attachment, having a linear or branched acyclic structure, at least one non-aromatic carbon-carbon double bond, no carbon-carbon triple bonds, and no atoms other than carbon and hydrogen. Non-limiting examples include: -CH=CH₂ (vinyl), -CH=CHCH₃, -CH=CHCH₂CH₃, -CH₂CH=CH₂ (allyl), - CH₂CH=CHCH₃, and -CH=CHCH=CH₂. The term "alkenediyl" as used without the modifier "substituted" represents a divalent unsaturated aliphatic group having 2 carbon atoms as the point of attachment, having a linear or branched, linear or branched acyclic structure, at least one non-aromatic carbon-carbon double bond, no carbon-carbon triple bonds, and no atoms other than carbon and hydrogen. The groups -CH=CH-, -CH=C(CH₃)CH₂-, -CH=CHCH₂-, and - CH₂CH=CHCH₂- are non-limiting examples of an alkenediyl group. It should be noted that although the alkenediyl group is aliphatic, once attached at both ends, it is not excluded that the group forms part of an aromatic structure. The terms "olefin" and "chain olefin" are synonymous and represent the class of compounds having formula H-R, wherein R is alkenyl, which is as defined above. Similarly, the terms "terminal olefin" and "α-olefin" are synonymous and represent olefin having exactly one carbon-carbon double bond, wherein the bond is part of vinyl at the terminus of the molecule. When any of these terms is used together with the modifier "substituted", one or more hydrogen atoms have been independently replaced by -OH, -F, -Cl, - Br, -I, -NH₂, -NO₂, -CO₂H, -CO₂CH₃, -CN, -SH, -OCH₃, -OCH₂CH₃, -C(O)CH₃, -NHCH₃, - NHCH₂CH₃, -N(CH₃)₂, -C(O)NH₂, -C(O)NHCH₃, -C(O)N(CH₃)₂, -OC(O)CH₃, -NHC(O)CH₃, - S(O)₂OH, or -S(O)₂NH₂. The groups -CH=CHF, -CH=CHCl, and -CH=CHBr are non-limiting examples of substituted alkenyl.

The term "alkynyl" as used without the modifier "substituted" represents a monovalent unsaturated aliphatic group having a carbon atom as the point of attachment, having a linear or branched acyclic structure, at least one carbon-carbon triple bond, and no atoms other than carbon and hydrogen. The term alkynyl as used herein does not preclude the presence of one or more non-aromatic carbon-carbon double bonds. The groups -C≡CH, -C≡CCH₃, and -CH₂C≡CCH₃ are non-limiting examples of alkynyl. "Alkyne" represents the class of compounds having formula H-R, wherein R is alkynyl. When any of these terms is used together with the modifier "substituted", one or more hydrogen atoms have been independently replaced by -OH, -F, -Cl, - Br, -I, -NH₂, -NO₂, -CO₂H, -CO₂CH₃, -CN, -SH, -OCH₃, -OCH₂CH₃, -C(O)CH₃, -NHCH₃, - NHCH₂CH₃, -N(CH₃)₂, -C(O)NH₂, -C(O)NHCH₃, -C(O)N(CH₃)₂, -OC(O)CH₃, -NHC(O)CH₃, - S(O)₂OH, or -S(O)₂NH₂.

"Prevention" or "preventing" includes: (1) inhibiting the onset of a disease in a subject or patient who may be at risk of and/or susceptible to the disease, but who has not yet experienced or exhibited any or all of the conditions or symptoms of the disease; and/or (2) slowing the onset of the conditions or symptoms of a disease in a subject or patient who may be at risk of and/or susceptible to the disease, but who has not experienced or exhibited any or all of the conditions or symptoms of the disease.

"Treatment" or "treating" includes: (1) inhibiting a disease (e.g., arresting the further development of the conditions and/or symptoms) in a subject or patient experiencing or exhibiting the conditions or symptoms of the disease, (2) ameliorating a disease (e.g., reversing the conditions and/or symptoms) in a subject or patient experiencing or exhibiting the conditions or symptoms of the disease, and/or (3) effecting any measurable mitigation of a disease in a subject or patient experiencing or exhibiting the conditions or symptoms of the disease.

"Protein", "polypeptide" or "peptide" refers to a polymer of amino acid residues, including a wide range of protein molecules such as cytokines, chemokines, interleukins, interferons, growth factors, coagulation factors, anticoagulants, blood factors, bone morphogenic proteins, immunoglobulins, and enzymes.

Antigens (abbreviated as Ag) refer to substances capable of causing antibody production and are any substances capable of inducing an immune response. Foreign molecules may be recognized by immunoglobulin on B cells, or treated by antigen-presenting cells, combined with a major histocompatibility complex to obtain a complex to reactivate T cells and trigger a continuous immune response.

An antigenic epitope, also known as an antigenic determinant, may be a special chemical group which consists of a continuous sequence (a protein primary structure) or a discontinuous protein three-dimensional structure and determines antigenicity. Most of antigenic epitopes are present on the surface of antigenic materials, and some of the antigenic epitopes are present inside the antigenic materials, and are exposed after treatment with enzymes or other means. One natural antigenic substance may have a variety of and a plurality of determinants. The larger the molecule of an antigen, the greater the number of the epitopes.

Tumor-associated antigens (TAA) refer to antigen molecules present on tumor cells or normal cells, including: embryonic proteins, glycoprotein antigens, squamous cell antigens, and the like, and are commonly used for clinical diagnosis of tumors. The tumor-associated antigens are not specific to the tumor cells, may be synthesized in minute amounts on the normal cells, are highly expressed when the tumor cells proliferate, and are therefore referred to as "associated antigens". Tumors derived from the same tissue type have the same tumor-associated antigens in different individuals.

Pathogenic microbes, also known as pathogens, refer to microbes that can invade human bodies and cause infections and even infectious diseases. Bacteria and viruses are the most harmful of antigens. Pathogenic microbes refer to prions, fungi, bacteria, spirochetes, mycoplasmas, rickettsias, chlamydias, and viruses. Pathogenic microbial antigens refer to substances that are derived from pathogens and have the function of triggering an immune response.

"Lipid encapsulation" refers to lipid nanoparticles which provide an active or a therapeutic agent (e.g., a nucleic acid (e.g., mRNA)), and comprise full encapsulation and partial encapsulation, or both. In some embodiments, the nucleic acid (e.g., mRNA) is completely encapsulated in the lipid nanoparticle.

In various embodiments, the lipid nanoparticle has an average diameter of: about 90 nm to about 600 nm, about 100 nm to about 550 nm, about 150 nm to about 500 nm, about 200 nm to about 400 nm, about 250 nm to about 300 nm, or about 200 nm to about 300 nm, and is substantially non-toxic.

### B. Helper lipid

In some aspects of the present disclosure, a composition containing one or more helper lipids is mixed with the cationic lipid disclosed herein to produce a lipid nanoparticle. In some embodiments, the cationic lipid is mixed with 1, 2, 3, 4, or 5 different types of helper lipids. It is contemplated that the cationic lipid is mixed with a single type of a variety of different helper lipids. In some embodiments, the helper lipid comprises, but is not limited to, one or more of a phospholipid, a steroid or a steroid derivative, a polymer conjugated lipid and a modified lipid.

"Phospholipid" is any lipid comprising a phosphoester group. In some embodiments, the phospholipid is a structure containing one or two long chain C6-C24 alkyl or alkenyl groups, glycerol or sphingosine, one or two phosphoester groups, and optionally a small organic molecule. In some embodiments, the small organic molecule is an amino acid, sugar, or an amino-substituted alkoxy group, such as choline or ethanolamine. In some embodiments, the phospholipid is phosphatidyl choline. In some embodiments, the phospholipid is DOPE, DSPC, DPPC, DMPC, DOPC, POPC or SM. In some embodiments, the phospholipid is DOPE or DSPC.

"Steroid and the steroid derivative" comprise any steroid or steroid derivative. As used herein, in some embodiments, the term "steroid" is a class of compounds having a tetracyclic 17-carbon cyclic structure, which can further contain one or more substitutions, wherein the substitutions include alkyl, alkoxy, hydroxy, oxo and acyl or double bonds between two or more carbon atoms. In one aspect, the ring structure of the steroid comprises three fused cyclohexyl rings and a fused cyclopentyl ring. In some embodiments, the steroid derivative comprises the above ring structure with one or more non-alkyl substitutions. In some embodiments, the steroid or the steroid derivative is a sterol. In some embodiments of the present disclosure, the steroid or the steroid derivative is a cholestane or a cholestane derivative. As described above, the cholestane derivative comprises one or more non-alkyl substitutions of the above ring system. In some embodiments, the cholestane or the cholestane derivative is a cholestene or a cholestene derivative, or a sterol or a sterol derivative. In other embodiments, the cholestane or the cholestane derivative is a cholestene and a sterol or a derivative thereof.

"Polymer conjugated lipid" refers to a lipid that inhibits aggregation of lipid nanoparticles or improves the stability of lipid nanoparticles or alters the immune response or alters the circulation time *in vivo.* The polymer conjugated lipid includes, but is not limited to, a polyethylene glycol conjugated lipid, a polylactic acid conjugated lipid, a polyamide conjugated lipid, a cationic polymer conjugated lipid, a poly-sarcosine (pSar) conjugated lipid, a poly(lactic-co-glycolic acid) (PLGA) conjugated lipid, a polyamino acid conjugated lipid, a polypeptide conjugated lipid, and a polypeptoid conjugated lipid, or a mixture thereof. In some embodiments, the "polyethylene glycol conjugated lipid" refers to any lipid to which a PEG group has been connected. In some embodiments, the PEG lipid is a diglyceride, also comprising a PEG chain connected to a glycerol group. In other embodiments, the PEG lipid is a compound containing one or more C6-C24 long chain alkyl or alkenyl groups or C6-C24 fatty acid groups connected to a linker group via a PEG chain. Some non-limiting examples of the PEG lipid include PEG-modified phosphatidylethanolamine and phosphatidic acid, PEG-conjugated ceramide, PEG-modified dialkylamine, PEG-modified 1,2-diacyloxypropane-3-amine, and PEG-modified diacylglycerol and dialkylglycerol. In some embodiments, the PEG lipid is PEG-modified distearoyl phosphatidylethanolamine or PEG-modified dimyristoyl-sn-glycerol. In some embodiments, PEG modification is measured with the molecular weight of the PEG component of the lipid. In some embodiments, the PEG used for modification has a molecular weight of about 100 to about 15000. In some embodiments, the molecular weight is about 200 to about 500, about 400 to about 5000, about 500 to about 3000, or about 1200 to about 3000. The molecular weight of the PEG used for modification is about 100, 200, 400, 500, 600, 800, 1000, 1250, 1500, 1750, 2000, 2250, 2500, 2750, 3000, 3500, 4000, 4500, 5000, 6000, 7000, 8000, 9000, 10000 or 12500 to about 15000. "Modified lipid" includes lipids modified by small-molecule compounds, vitamins, carbohydrates, peptides, proteins, nucleic acids, lipopolysaccharides, inorganic molecules or particles, metal ions or particles, and combinations of the substances described above.

In addition, throughout the specification and claims, unless explicitly stated otherwise, the term "comprise", or variations such as "comprises" or "comprising", will be understood to imply the inclusion of the stated element or component but not the exclusion of any other element or component.

### English abbreviation list

| Abbreviation | English | |
|---|---|---|
| siRNA | small interference RNA | |
| saRNA | small activating RNA | |
| samRNA | self-amplifying mRNA | |
| miRNA | microRNA | |
| aiRNA | asymmetric interfering RNA | |
| dsRNA | Dicer-substrate RNA | |
| shRNA | small hairpin RNA | |
| DODAP | *N*-[2,3-Di(oleoyloxy)propyl]-*N*,*N*-dimethylamine | |
| ALC-0315 | 2-hexyl-decanoic acid, 1,1'-[[(4-hydroxybutyl)imino]di-6,1-hexanediyl] ester | |
| DOPE | 1,2-dioleoyl-sn-glycero-3-phosphoethanolamine | |
| DSPC | 1,2-distearoyl-sn-glycero-3-phosphocholine | |
| DPPC | 1,2-dipalmitoyl-sn-glycero-3-phosphocholine | |
| DMPC | 1,2-dimyristoyl-sn-glycero-3-phosphocholine | |
| DOPC | 1,2-dioleoyl-sn-glycero-3-phosphocholine | |
| POPC | 1-palmitoyl-2-oleoyl-sn-glycero-3-phosphocholine | |
| PEG2000-DMG | 1,2-Dimyristoyl-rac-glycero-3-methoxypolyethylene glycol | |

In order to more clearly describe the objects, features, and advantages of the present disclosure, the present disclosure will be described below in detail with reference to the drawings and specific embodiments, but the embodiments of the present disclosure described below in detail are intended only to illustrate the content of the present disclosure and do not constitute any limitation to the present disclosure.

In the specific embodiments of the present disclosure, the starting materials used can be commercially available.

### Example 1: Synthesis of Compound

Compound 1 was synthesized according to the following reaction scheme:

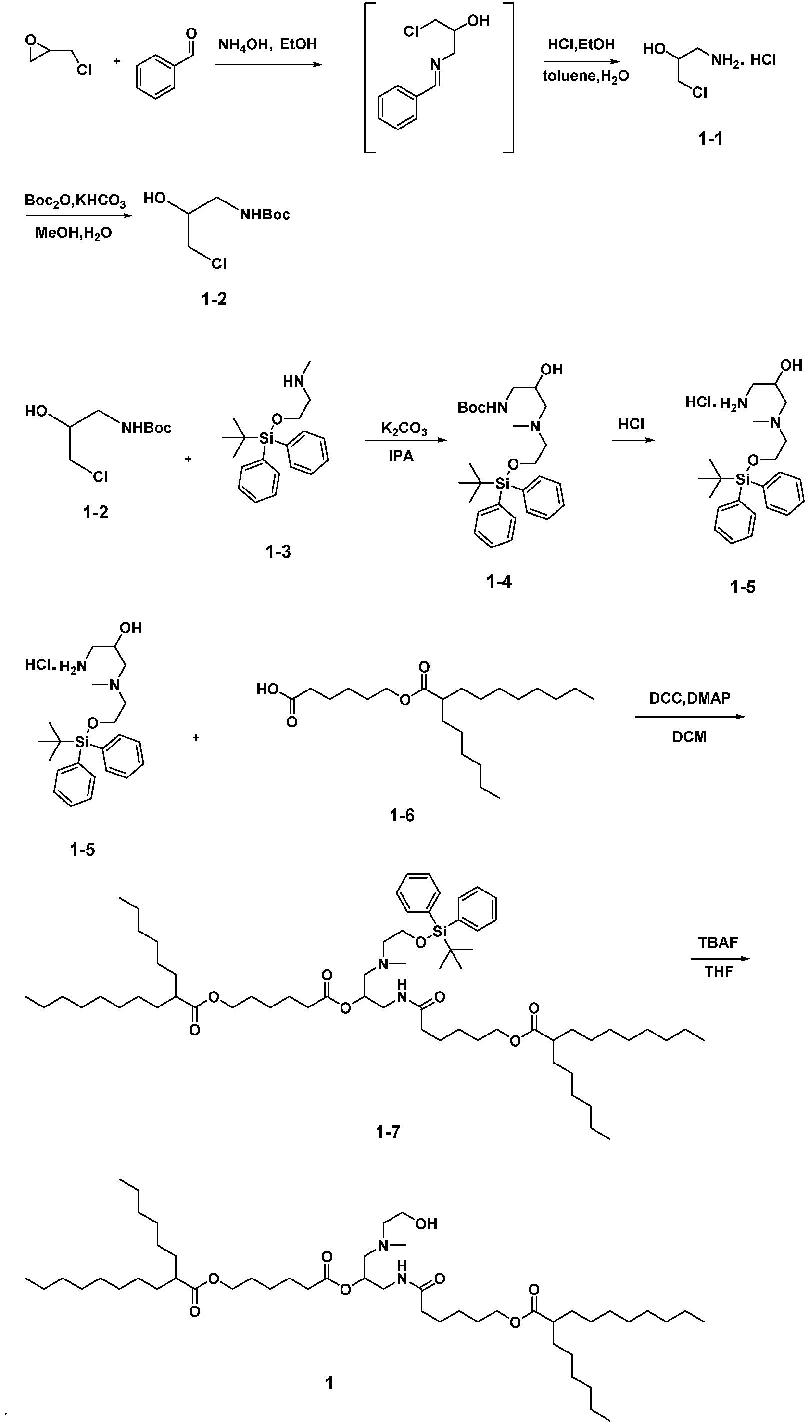

### Step 1: synthesis of compound 1-1

Benzaldehyde (5.91 g, 55.66 mmol) and ethanol (17 mL) were added into a 100 mL flask, and the solution was cooled to 18 °C. Ammonium hydroxide (11.97 g, 85.39 mmol) was slowly added dropwise to the solution, and after the dropwise addition, the mixture was stirred at 18 °C for 2 h. Epichlorohydrin (5 g, 54.04 mmol) was then weighed out, and the mixture was stirred at 40-45 °C for 16 h. After completion of the reaction, the reaction solution was concentrated to dryness, and toluene (20 mL) and water (20 mL) were added to dissolve the crude product. Concentrated hydrochloric acid (8.32 g, 82.13 mmol) was then weighed out and added dropwise into the reaction system, and the mixture was stirred for 3 h with the temperature maintained at 35-40 °C, followed by phase separation. The toluene phase was washed with water (10 mL). The aqueous phases were combined and concentrated to obtain a product (compound 1-1, 6 g).

### Step 2: synthesis of compound 1-2

Compound 1-1 (3 g, 20.55 mmol), di-*tert*-butyl dicarbonate Boc₂O (4.89 g, 22.40 mmol), methanol (12 mL), and water (7 mL) were added into a 100 mL flask. Potassium bicarbonate (2.26 g, 11.60 mmol) was weighed out and added to the reaction solution in three batches, and the mixture was stirred for 3 h. The reaction progress was monitored by TLC (10% MeOH/DCM). After completion of the reaction, dichloromethane (15 mL) was added, and the mixture was extracted with water (6 mL), followed by phase separation. The organic phase was concentrated to obtain a crude product (about 5 g). The crude product was purified and separated using a Flash column (20 g silica gel; 100 mL of *n*-heptane; 100 mL of 1% EtOAc + 99% *n-*heptane; 100 mL of 5% EtOAc + 95% n-heptane; 600 mL of 10% EtOAc + 90% *n*-heptane) to obtain a pure product (compound 1-2, 800 mg, 19% yield). ¹H NMR (400 MHz, CDCl₃): *δ* 4.94 (m, 1H), 3.81-3,75 (m, 1H), 3.73-3.69 (m, 1H), 3.50-3.46 (m, 1H), 3.25-3.20 (m, 2H), 3.00-2.94 (m, 2H), 2.63-2.56 (m, 2H), 2.53-2.48 (m, 1H), 2.37-2.33 (m, 1H), 2.31 (m, 3H), 1.43 (s, 9H).

### Step 3: synthesis of compound 1-4

Compound 1-2 (700 mg, 3.34 mmol), N-methyl-2-(*tert*-butyldiphenylsilyloxy) ethylamine (compound 1-3, CAS: 165689-59-8) (1.05 g, 3.34 mmol), potassium carbonate (1.15 g, 8.35 mmol), and isopropanol IPA (8 mL) were added into a 25 mL flask. The mixture was heated to 80 °C and stirred for 16 h. The reaction progress was monitored by TLC (10% MeOH/DCM). After completion of the reaction, the mixture was filtered, and the organic phase was concentrated to obtain a crude product (about 2 g). The crude product was purified and separated using a Flash column (12 g silica gel; the mobile phases were respectively: 100 mL of *n*-heptane; 100 mL of 1% EtOAc + 99% n-heptane; 100 mL of 2% EtOAc + 98% *n*-heptane; 100 mL of 8% EtOAc + 92% n-heptane; 100 mL of 15% EtOAc + 85% n-heptane; 600 mL of 30% EtOAc + 70% n-heptane) to obtain a pure product (compound 1-4, 1 g, 62% yield). ¹H NMR (400 MHz, CDCl₃): *δ* 7.68-7.66 (m, 4H), 7.43-7.37 (m, 6H), 4.99 (m, 1H), 3.74-3,70 (m, 3H), 3.35-3.31 (m, 1H), 3.03-2.97 (m, 1H), 2.73-2.67 (m, 1H), 2.55-2.52 (m, 1H), 2.41-2.38 (m, 2H), 2.30 (m, 3H), 1.44 (s, 9H), 1.06-1.04 (m, 9H).

### Step 4: synthesis of compound 1-5

Compound 1-4 (300 mg, 0.62 mmol) and ethyl acetate (2 mL) were added into a 25 mL flask, and the mixture was cooled in an ice bath to 0-5 °C. HCl/EtOAc (0.6 mL, 4 M) was then weighed out and added, and the mixture was warmed to room temperature and stirred for 3 h. The reaction progress was monitored by TLC (10% MeOH/DCM). After completion of the reaction, the organic phase was concentrated to obtain a product (compound 1-5, about 300 g).

### Step 5: synthesis of compound 1-7

5-Carboxypentyl 2-hexyldecanoate (compound 1-6, CAS: 2805989-43-7) (496.14 mg, 1.34 mmol), 1,3-dicyclohexylcarbodiimide DCC (276.25 mg, 1.34 mmol), 4-dimethylaminopyridine DMAP (122.68 mg, 1.0 mmol), and dichloromethane DCM (5 mL) were added into a 25 mL flask, and the mixture was stirred at room temperature for 30 min. Compound 1-5 (330 mg, 0.66 mmol) was then added dropwise, and the mixture was stirred at room temperature for 16 h. The reaction progress was monitored by TLC (30% EtOAc/*n*-heptane). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 1 g). The crude product was purified and separated using a Flash column (12 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 200 mL of 20% EtOAc + 80% n-heptane) to obtain a pure product (compound 1-7, 330 mg, 45% yield). ¹H NMR (400 MHz, CDCl₃): *δ* 7.67-7.65 (m, 4H), 7.45-7.36 (m, 6H), 6.26 (m, 1H), 4.98 (m, 1H), 4.07-4.02 (m, 4H), 3.75 (m, 2H), 3.49-3.45 (m, 2H), 2.59 (m, 4H), 2.34-2.22 (m, 7H), 2.11-2.08 (m, 2H), 1.67-1.53 (m, 14H), 1.46-1.25 (m, 60H), 1.05 (s, 9H), 0.89-0.85 (m, 12H).

### Step 6: synthesis of compound 1

Compound 1-7 (330 mg, 0.31 mmol) and tetrahydrofuran THF (3 mL) were added into a 25 mL flask, and the mixture was cooled in an ice bath to 0-5 °C. tetrabutylammonium fluoride TBAF (240.19 mg, 0.92 mmol) was added dropwise with the temperature maintained at 0-5 °C, and the mixture was stirred at this temperature for 3 h. The reaction progress was monitored by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to obtain a crude product (about 500 mg). The crude product was purified and separated using a Flash column (4 g silica gel, loading with 100 mL of n-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; 300 mL of 50% EtOAc + 50% *n*-heptane) to obtain a pure product (compound 1, 100 mg, 39% yield). ¹H NMR (400 MHz, CDCl₃): *δ* 6.14-6.11 (m, 1H), 5.06-5.04 (m, 1H), 4.08-4.04 (m, 4H), 3.63-3.46 (m, 4H), 2.64-2.60 (m, 4H), 2.35-2.26 (m, 7H), 2.20-2.17 (m, 2H), 1.70-1.53 (m, 12H), 1.46-1.35 (m, 8H), 1.33-1.25 (m, 45H), 0.89-0.85 (m, 12H).

Compound 2 was synthesized according to the following reaction scheme:

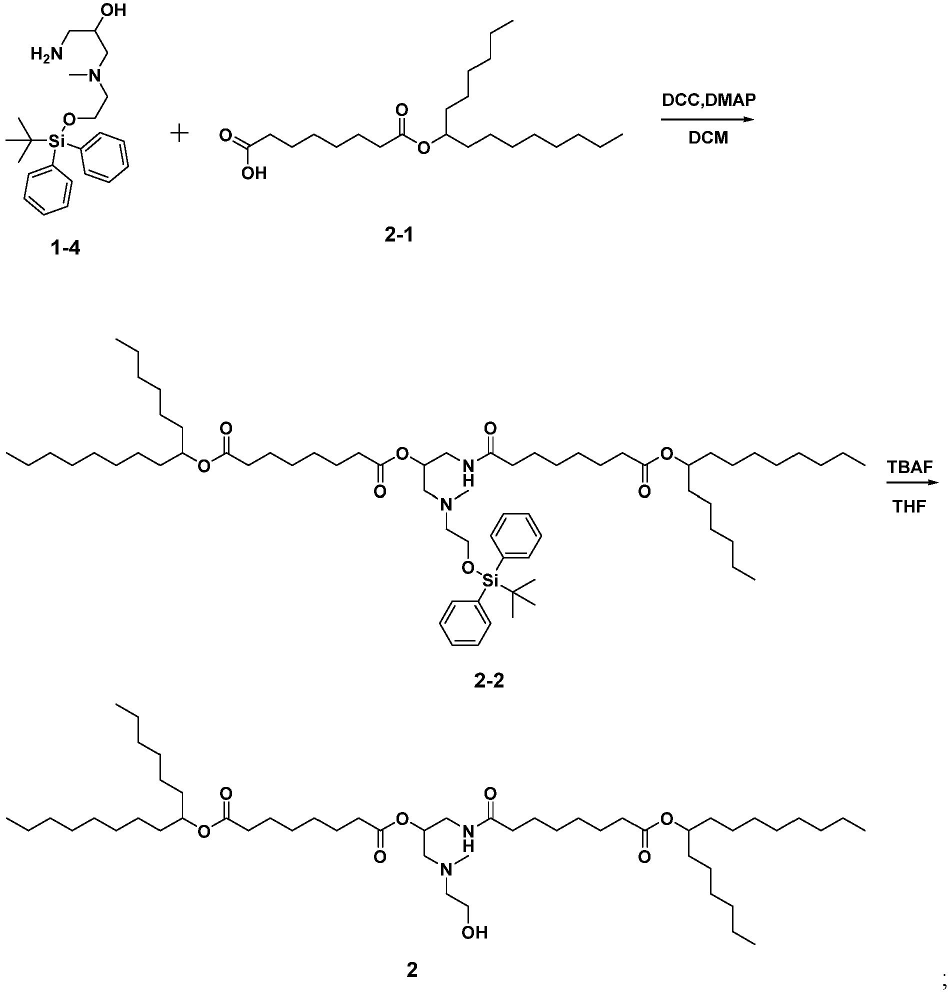

### Step 1: synthesis of compound 2-2

8-Oxo-8-(pentadecan-7-yloxy)octanoic acid (compound 2-1, CAS: 2765518-33-8) (840.64 mg, 2.19 mmol), 1,3-dicyclohexylcarbodiimide DCC (450.99 mg, 2.19 mmol), 4-dimethylaminopyridine DMAP (133.52 mg, 1.09 mmol), and dichloromethane DCM (3 mL) were added into a 25 mL flask, and the mixture was stirred at room temperature for 30 min. Compound 1-4 (500 mg, 1.09 mmol) was then added dropwise, and the mixture was stirred at room temperature overnight. The reaction progress was monitored by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 1.6 g). The crude product was purified and separated using a Flash column (12 g silica gel; the mobile phases were respectively: 100 mL of *n*-heptane; 100 mL of 1% EtOAc + 99% *n*-heptane; 100 mL of 2% EtOAc + 98% *n-*heptane; 100 mL of 8% EtOAc + 92% *n*-heptane) to obtain a pure product (compound 2-2, 320 mg, 25% yield).

### Step 2: synthesis of compound 2

Compound 2-2 (320 mg, 0.29 mmol) and tetrahydrofuran THF (3 mL) were added into a 25 mL flask, and the mixture was cooled in an ice bath to 0-5 °C. Tetrabutylammonium fluoride TBAF (224.16 mg, 0.86 mmol) was added dropwise with the temperature maintained at 0-5 °C, and the mixture was stirred at this temperature for 3 h. The reaction progress was monitored by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to obtain a crude product (about 500 mg). The crude product was purified and separated using a Flash column (4 g silica gel, loading with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; 300 mL of 50% EtOAc + 50% *n*-heptane) to obtain a pure product (compound 2, 100 mg, 40% yield). ¹H NMR (400 MHz, CDCl₃) δ 6.09 (d, *J =* 6.4 Hz, 1H), 5.05 (q, *J =* 5.7 Hz, 1H), 4.85 (p, *J =* 6.2 Hz, 2H), 3.66-3.41 (m, 4H), 2.61 (t, *J =* 5.6 Hz, 4H), 2.39-2.22 (m, 9H), 2.20-2.12 (m, 2H), 1.67-1.44 (m, 17H), 1.40-1.16 (m, 53H), 0.87 (t, *J =* 6.7 Hz, 12H).

Compound 3 was synthesized according to the following reaction scheme:

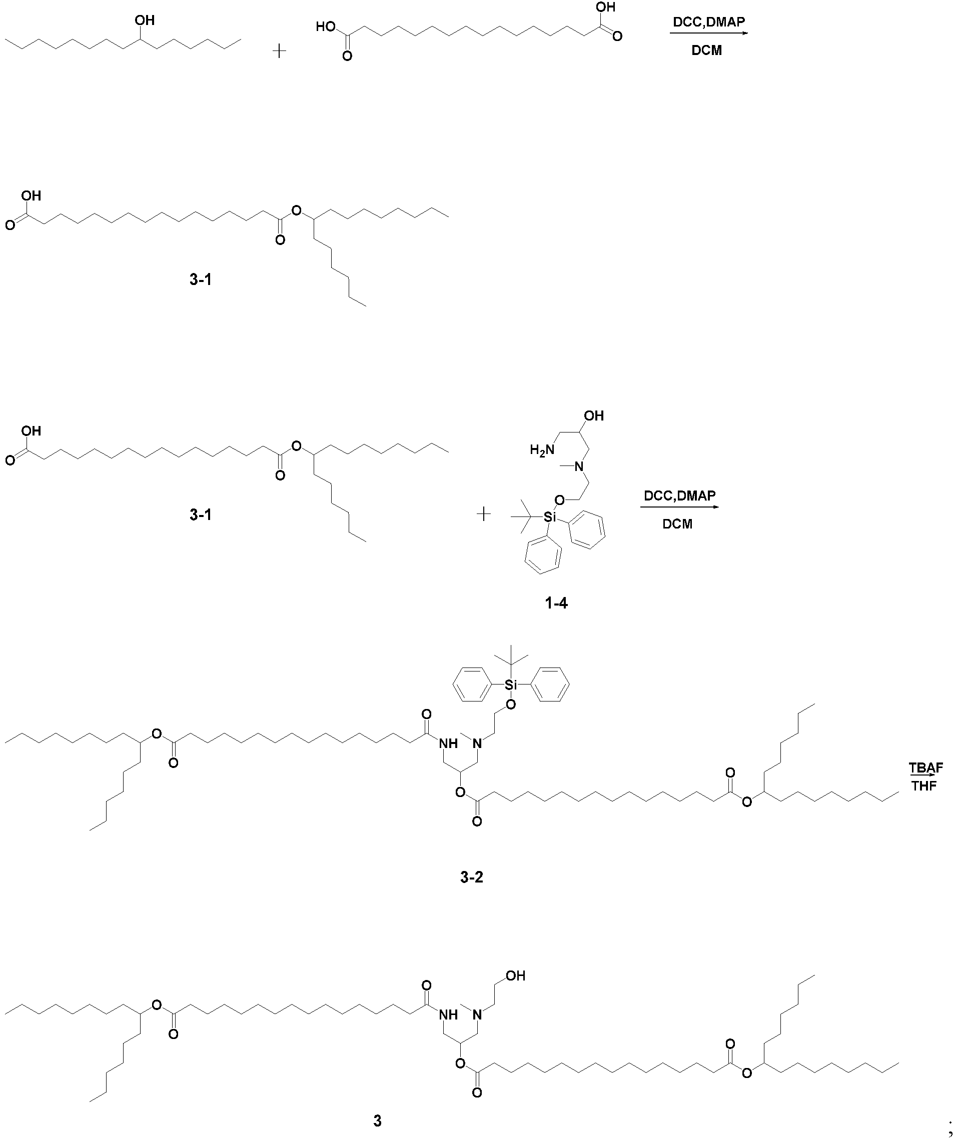

### Step 1: synthesis of compound 3-1

Hexadecanedioic acid (5.64 g, 19.7 mmol), 1,3-dicyclohexylcarbodiimide DCC (4.06 g, 19.7 mmol), 4-dimethylaminopyridine DMAP (802.28 mg, 6.57 mmol), and dichloromethane DCM (30 mL) were added into a 250 mL flask, and the mixture was stirred at room temperature for 30 min. Pentadecan-7-ol (1.5 g, 6.57 mmol) was then added dropwise, and the mixture was stirred at room temperature overnight. The reaction progress was monitored by TLC (30% ethyl acetate/*n*-heptane). After completion of the reaction, the mixture was filtered, and the filtrate was washed with 50 mL of water and 50 mL of saturated brine, dried over anhydrous Na₂SO₄, and filtered. The organic phase was concentrated to obtain a crude product (about 5 g). The crude product was purified and separated using a Flash column (40 g silica gel; the mobile phases were respectively: 100 mL of *n*-heptane; 100 mL of 1% EtOAc + 99% *n*-heptane; 100 mL of 2% EtOAc + 98% *n*-heptane; 800 mL of 8% EtOAc + 92% *n*-heptane) to obtain a pure product (compound 3-1, 1.4 g, 31% yield). ¹H NMR (400 MHz, CDCl₃) δ 4.86 (p, *J =* 6.3 Hz, 1H), 2.31 (dt, *J =* 26.9, 7.5 Hz, 4H), 1.62 (h, *J =* 7.1 Hz, 4H), 1.50 (q, *J =* 6.1 Hz, 4H), 1.40-1.16 (m, 42H), 0.87 (t, *J =* 6.7 Hz, 6H).

### Step 2: synthesis of compound 3-2

Compound 3-1 (706.27 mg, 1.42 mmol), 1,3-dicyclohexylcarbodiimide DCC (293.32 mg, 1.42 mmol), 4-dimethylaminopyridine DMAP (86.64 mg, 0.72 mmol), and dichloromethane DCM (30 mL) were added into a 25 mL flask, and the mixture was stirred at room temperature for 30 min. Compound 1-4 (derived from an intermediate in the synthesis of compound 1 described above) (300 mg, 0.72 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 1.2 g). The crude product was purified and separated using a Flash column (4 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; 400 mL of 30% EtOAc + 70% n-heptane) to obtain a pure product (compound 3-2, 240 mg, 25% yield). ¹H NMR (400 MHz, CDCl₃) δ 7.72-7.61 (m, 5H), 7.48-7.33 (m, 8H), 4.98 (s, 1H), 4.87 (p, *J =* 6.3 Hz, 3H), 3.74 (s, 2H), 3.47 (t, *J =* 5.8 Hz, 3H), 2.73-2.49 (m, 4H), 2.27 (t, *J =* 7.5 Hz, 12H), 1.70-1.44 (m, 23H), 1.26 (td, *J =* 7.4, 6.3, 3.0 Hz, 124H), 1.05 (s, 9H), 0.91-0.84 (m, 12H).

### Step 3: synthesis of compound 3

Compound 3-2 (240 mg, 0.18 mmol) and tetrahydrofuran THF (3 mL) were added into a 25 mL flask, and the mixture was cooled in an ice bath to 0-5 °C. tetrabutylammonium fluoride TBAF (140.05 mg, 0.54 mmol) was added dropwise with the temperature maintained at 0-5 °C, and the mixture was stirred at this temperature for 3 h. The reaction progress was monitored by TLC (5% MeOH/DCM). After completion of the reaction, the reaction was quenched with a saturated aqueous ammonium chloride solution, and the mixture was extracted with EtOAc (10 mL × 2). The organic phases were combined, dried over anhydrous Na₂SO₄, filtered, and concentrated to obtain a crude product (about 250 mg). The crude product was purified and separated using a Flash column (4 g silica gel, loading with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; 300 mL of 50% EtOAc + 50% *n*-heptane) to obtain a pure product (compound 3, 80 mg, 40% yield). ¹H NMR (400 MHz, CDCl₃) δ 5.09 (s, 1H), 4.86 (p, *J =* 6.3 Hz, 2H), 3.64 (t, *J =* 5.2 Hz, 2H), 3.50 (ddd, *J =* 14.3, 8.9, 5.5 Hz, 2H), 2.67 (s, 3H), 2.44-2.11 (m, 12H), 1.61 (p, *J* = 7.3 Hz, 9H), 1.50 (q, *J* = 6.1 Hz, 8H), 1.35-1.17 (m, 96H), 0.87 (t, *J =* 6.7 Hz, 12H).

Compound 4 was synthesized according to the following reaction scheme:

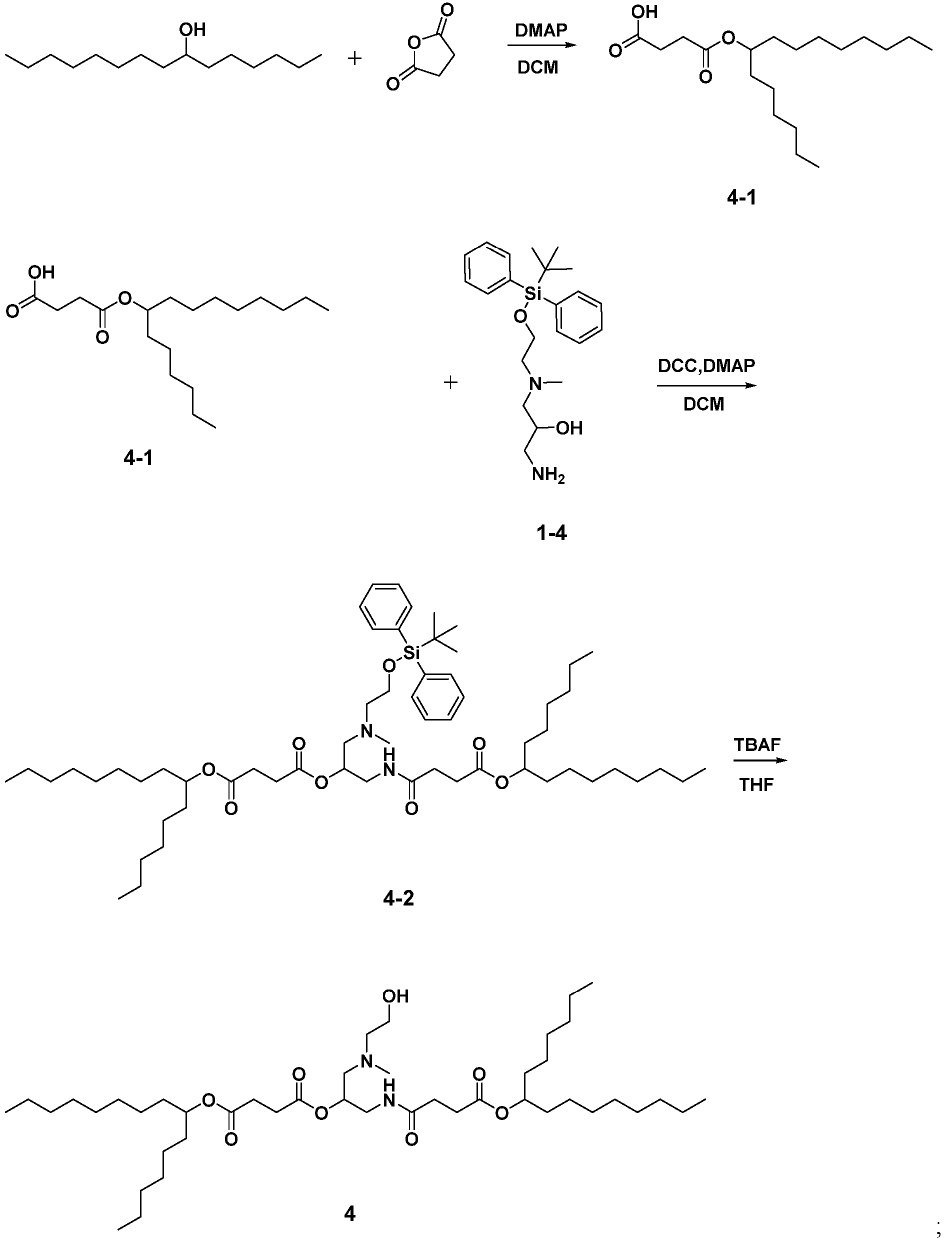

### Step 1: synthesis of compound 4-1

Succinic anhydride (1.75 g, 17.51 mmol), 4-dimethylaminopyridine DMAP (2.14 g, 17.51 mmol), pentadecan-7-ol (1.5 g, 6.57 mmol), and dichloromethane DCM (30 mL) were added into a 250 mL flask, and the mixture was stirred at room temperature overnight. The reaction progress was monitored by TLC (30% ethyl acetate/*n*-heptane). After completion of the reaction, the mixture was filtered, and the filtrate was washed with 50 mL of water and 50 mL of saturated brine, dried over anhydrous Na₂SO₄, and filtered. The organic phase was concentrated to obtain a crude product (about 5 g). The crude product was purified and separated using a Flash column (40 g silica gel; the mobile phases were respectively: 100 mL of *n*-heptane; 100 mL of 1% EtOAc + 99% *n*-heptane; 100 mL of 2% EtOAc + 98% *n*-heptane; 600 mL of 8% EtOAc + 92% *n*-heptane) to obtain a pure product (compound 4-1, 2.4 g, 42% yield). ¹H NMR (400 MHz, CDCl₃) δ 4.88 (p, *J =* 6.3 Hz, 1H), 2.74-2.55 (m, 4H), 1.51 (q, *J =* 6.2 Hz, 4H), 1.26 (d, *J =* 6.0 Hz, 22H), 0.87 (t, *J =* 6.7 Hz, 6H).

### Step 2: synthesis of compound 4-2

Compound 4-1 (466.98 mg, 1.42 mmol), 1,3-dicyclohexylcarbodiimide DCC (293.32 mg, 1.42 mmol), 4-dimethylaminopyridine DMAP (86.64 mg, 0.72 mmol), and dichloromethane DCM (3 mL) were added into a 25 mL flask, and the mixture was stirred at room temperature for 30 min. Compound 1-4 (derived from an intermediate in the synthesis of compound 1 described above) (300 mg, 0.72 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 800 mg). The crude product was purified and separated using a Flash column (4 g silica gel; the mobile phases were respectively: 100 mL of *n*-heptane; 100 mL of 1% EtOAc + 99% *n*-heptane; 100 mL of 2% EtOAc + 98% *n*-heptane; 800 mL of 8% EtOAc + 92% *n*-heptane; 300 mL of 20% EtOAc + 80% *n*-heptane) to obtain a pure product (compound 4-2, 240 mg, 33.5% yield). ¹H NMR (400 MHz, CDCl₃) δ 7.66 (d, *J =* 6.9 Hz, 5H), 7.41 (t, *J =* 8.6 Hz, 7H), 5.03 (s, 1H), 4.92-4.76 (m, 3H), 2.74-2.51 (m, 12H), 1.60-1.46 (m, 11H), 1.35-1.17 (m, 64H), 1.05 (s, 9H), 0.87 (t, *J* = 6.6 Hz, 12H).

### Step 3: synthesis of compound 4

Compound 4-2 (240 mg, 0.24 mmol) and tetrahydrofuran THF (3 mL) were added into a 25 mL flask, and the mixture was cooled in an ice bath to 0-5 °C. Tetrabutylammonium fluoride TBAF (186.84 mg, 0.72 mmol) was added dropwise with the temperature maintained at 0-5 °C, and the mixture was stirred at this temperature for 3 h. The reaction progress was monitored by TLC (5% MeOH/DCM). After completion of the reaction, the reaction was quenched with a saturated aqueous ammonium chloride solution, and the mixture was extracted with EtOAc (10 mL × 2). The organic phases were combined, dried over anhydrous Na₂SO₄, filtered, and concentrated to obtain a crude product (about 250 mg). The crude product was purified and separated using a Flash column (4 g silica gel, loading with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 300 mL of 30% EtOAc + 70% *n*-heptane) to obtain a pure product (compound 4, 80 mg, 44% yield). ¹H NMR (400 MHz, CDCl₃) δ 6.56 (s, 1H), 5.18-5.07 (m, 1H), 4.85 (dp, *J =* 12.3, 6.2 Hz, 2H), 3.61 (ddt, *J =* 10.4, 6.2, 3.8 Hz, 3H), 3.43 (dt, *J =* 14.1, 6.0 Hz, 1H), 2.83 - 2.55 (m, 10H), 2.55-2.33 (m, 5H), 1.59-1.42 (m, 8H), 1.26 (d, *J =* 6.7 Hz, 42H), 0.87 (t, *J =* 6.7 Hz, 12H).

Compound 5 was synthesized according to the following reaction scheme:

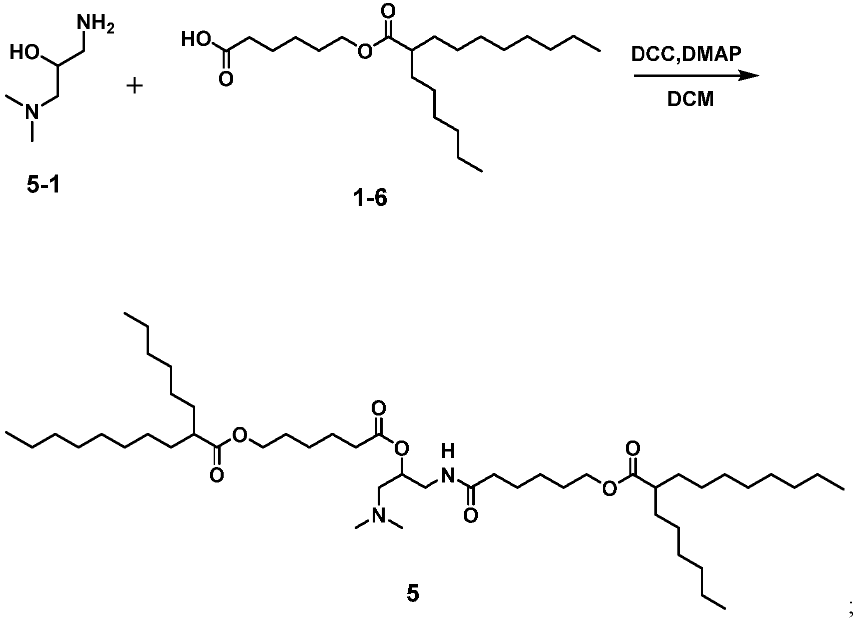

### Step 1: synthesis of compound 5

4-Dimethylaminopyridine DMAP (65.9 mg, 0.54 mmol), 1,3-dicyclohexylcarbodiimide DCC (222.7 mg, 1.1 mmol), and dichloromethane DCM (1.5 mL) were added into a 25 mL flask, and the mixture was stirred until it became clear. 5-Carboxypentyl 2-hexyldecanoate (compound 1-6, CAS: 2805989-43-7) (400 mg, 1.1 mmol) was added, and the mixture was stirred at room temperature for 30 min. 1-amino-3-(dimethylamino)propanol (compound 5-1, purchased from Bide Pharmatech Ltd., 63.8 mg, 0.55 mmol) was then added in one batch, and the mixture was stirred at room temperature overnight. The reaction progress was monitored by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain a crude product (about 510 mg). The crude product was purified and separated using a Flash column (20 g silica gel, loading with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; 100 mL of 20% EtOAc + 80% *n*-heptane; 300 mL of 50% EtOAc + 50% n-heptane) to obtain a pure product (compound 5, 300 mg, 67% yield). ¹H NMR (400 MHz, CDCl₃): *δ* 6.32-6.29 (m, 1H), 5.03-4.97 (m, 2H), 4.07-4.04 (m, 4H), 3.02 (m, 2H), 2.49-2.39 (m, 2H), 2.33-2.26 (m, 11H), 2.18-2.14 (m, 2H), 1.69-1.53 (m, 12H), 1.46-1.24 (m, 51H), 0.88-0.85 (m, 12H).

Compound 6 was synthesized according to the following reaction scheme:

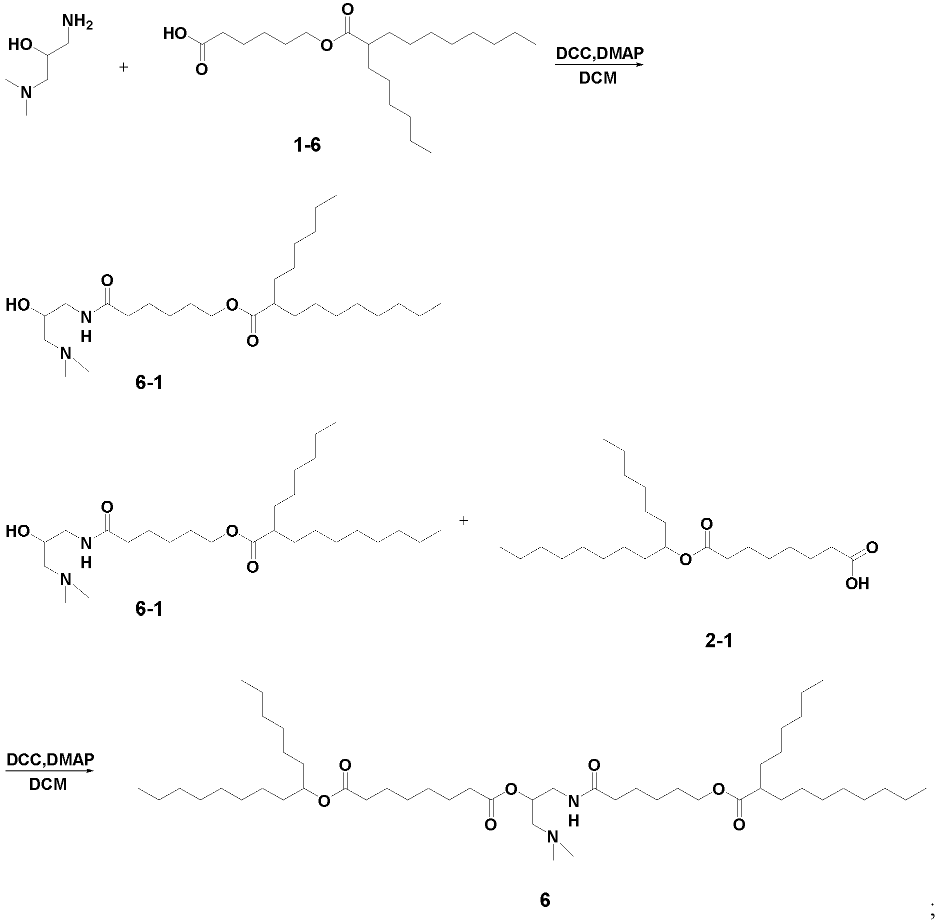

### Step 1: synthesis of compound 6-1

5-Carboxypentyl 2-hexyldecanoate (compound 1-6, CAS: 2805989-43-7) (2.1 g, 5.67 mmol), 1,3-dicyclohexylcarbodiimide DCC (1.17 g, 5.67 mmol), 4-dimethylaminopyridine DMAP (692.63 mg, 5.67 mmol), and dichloromethane DCM (10 mL) were added into a 25 mL flask, and the mixture was stirred at room temperature for 30 min. 1-Amino-3-(dimethylamino)propan-2-ol (670 mg, 5.67 mmol) was then added dropwise, and the mixture was stirred at room temperature overnight. The reaction progress was monitored by TLC (5% MeOH/DCM). After completion of the reaction, the mixture was filtered, and the filtrate was washed with 10 mL of water and 10 mL of saturated brine, dried over anhydrous Na₂SO₄, and filtered. The organic phase was concentrated to obtain a crude product (about 3.5 g). The crude product was purified and separated using a Flash column (40 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; 600 mL of 50% EtOAc + 50% *n*-heptane) to obtain a pure product (compound 6-1, 1 g, 37% yield).

### Step 2: synthesis of compound 6

8-Oxo-8-(pentadecan-7-yloxy)octanoic acid (compound 2-1, CAS: 2765518-33-8) (245.10 mg, 0.64 mmol), 1,3-dicyclohexylcarbodiimide DCC (131.50 mg, 0.64 mmol), 4-dimethylaminopyridine DMAP (77.86 mg, 0.64 mmol), and dichloromethane DCM (3 mL) were added into a 25 mL flask, and the mixture was stirred at room temperature for 30 min. Compound 6-1 (300 mg, 0.64 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 700 mg). The crude product was purified and separated using a Flash column (12 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; 700 mL of 30% EtOAc + 70% *n*-heptane) to obtain a pure product (compound 6, 240 mg, 45% yield). ¹H NMR (400 MHz, CDCl₃) δ 6.34 (d, *J =* 5.7 Hz, 1H), 5.01 (p, *J =* 5.9 Hz, 1H), 4.85 (p, *J =* 6.3 Hz, 1H), 4.05 (t, *J =* 6.7 Hz, 2H), 3.56-3.41 (m, 2H), 2.48 (q, *J =* 12.9, 6.1 Hz, 2H), 2.37-2.24 (m, 11H), 2.17 (t, *J* = 7.7 Hz, 2H), 1.73-1.15 (m, 66H), 0.87 (t, *J =* 6.7 Hz, 12H).

Compound 7 was synthesized according to the following reaction scheme:

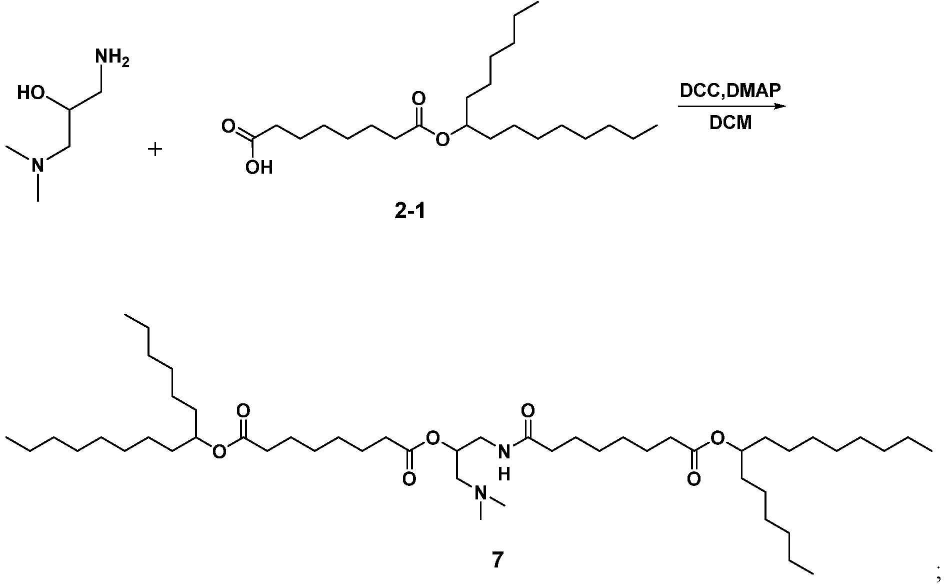

### Step 1: synthesis of compound 7

8-Oxo-8-(pentadecan-7-yloxy)octanoic acid (compound 2-1, CAS: 2765518-33-8) (650.87 mg, 1.69 mmol), 1,3-dicyclohexylcarbodiimide DCC (349.18 mg, 1.69 mmol), 4-dimethylaminopyridine DMAP (103.38 mg, 0.85 mmol), and dichloromethane DCM (5 mL) were added into a 25 mL flask, and the mixture was stirred at room temperature for 30 min. 1-Amino-3-(dimethylamino)propan-2-ol (100 mg, 0.85 mmol) was then added dropwise, and the mixture was stirred at room temperature overnight. The reaction progress was monitored by TLC (5% MeOH/DCM). After completion of the reaction, the mixture was filtered, and the filtrate was washed with 10 mL of water and 10 mL of saturated brine, dried over anhydrous Na₂SO₄, and filtered. The organic phase was concentrated to obtain a crude product (about 800 mg). The crude product was purified and separated using a Flash column (12 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; 800 mL of 30% EtOAc + 70% *n*-heptane) to obtain a pure product (compound 7, 460 mg, 64% yield). ¹H NMR (400 MHz, CDCl₃) δ 6.32 (s, 1H), 5.01 (t, *J* = 5.8 Hz, 1H), 4.85 (p, *J* = 6.2 Hz, 2H), 3.49 (t, *J =* 5.1 Hz, 2H), 2.47 (q, *J =* 12.8, 6.1 Hz, 2H), 2.36-2.23 (m, 12H), 2.15 (t, *J =* 7.6 Hz, 2H), 1.56 (d, *J =* 48.7, 6.5, 6.0 Hz, 17H), 1.40-1.16 (m, 53H), 0.87 (t, *J =* 6.7 Hz, 12H).

Compound 8 was synthesized according to the following reaction scheme:

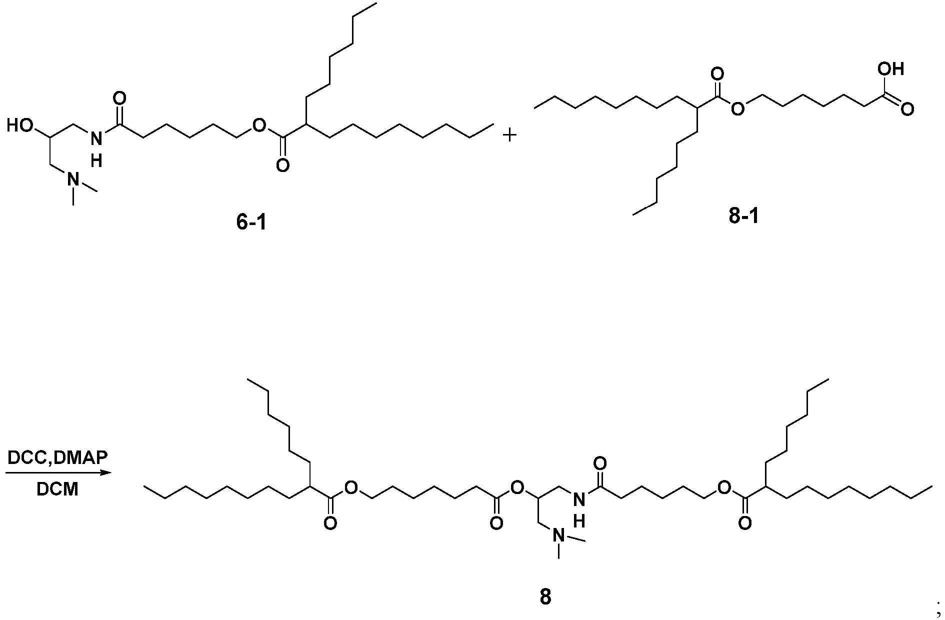

### Step 1: synthesis of compound 8

6-Carboxyhexyl 2-hexyldecanoate (compound 8-1, CAS: 2805989-46-0) (245.10 mg, 0.64 mmol), 1,3-dicyclohexylcarbodiimide DCC (131.50 mg, 0.64 mmol), 4-dimethylaminopyridine DMAP (77.86 mg, 0.64 mmol), and dichloromethane DCM (3 mL) were added into a 25 mL flask, and the mixture was stirred at room temperature for 30 min. Compound 6-1 (derived from an intermediate in the synthesis of compound 6 described above) (300 mg, 0.64 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 600 mg). The crude product was purified and separated using a Flash column (12 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 200 mL of 20% EtOAc + 80% n-heptane) to obtain a pure product (compound 8, 120 mg, 22% yield). ¹H NMR (400 MHz, CDCl₃) δ 6.34 (d, *J* = 5.5 Hz, 1H), 5.01 (t, *J* = 5.9 Hz, 1H), 4.05 (t, *J* = 6.7 Hz, 4H), 3.49 (t, *J* = 5.6 Hz, 2H), 2.48 (dd, *J* = 8.6, 6.2 Hz, 2H), 2.30 (d, *J* = 11.3 Hz, 10H), 2.17 (t, *J* = 7.7 Hz, 2H), 1.71-1.50 (m, 13H), 1.48-1.14 (m, 57H), 0.87 (t, *J* = 6.7 Hz, 12H).

Compound 9 was synthesized according to the following reaction scheme:

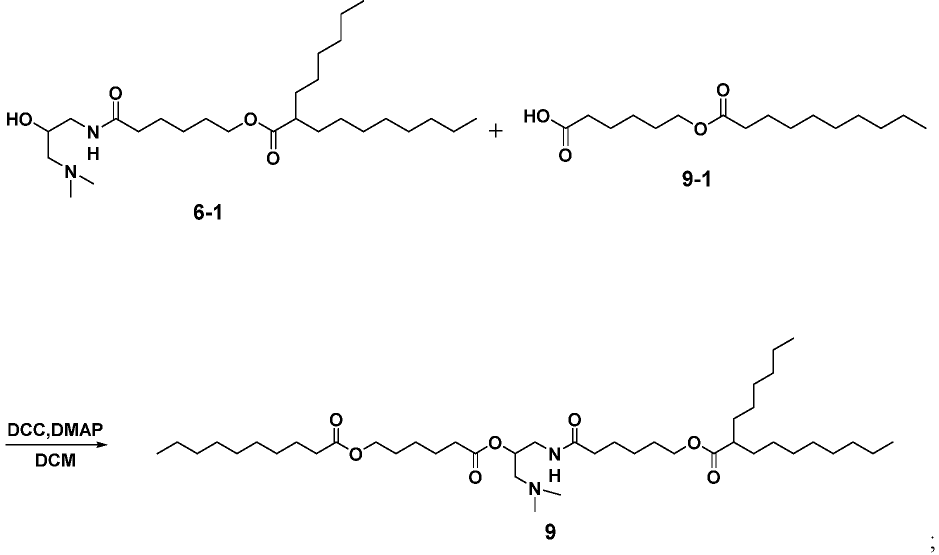

### Step 1: synthesis of compound 9

6-(Decanoyloxy)acid compound (compound 9-1, CAS: 2254439-01-3) (201.21 mg, 0.70 mmol), 1,3-dicyclohexylcarbodiimide DCC (144.96 mg, 0.70 mmol), 4-dimethylaminopyridine DMAP (85.83 mg, 0.70 mmol), and dichloromethane DCM (3 mL) were added into a 25 mL flask, and the mixture was stirred at room temperature for 30 min. Compound 6-1 (derived from an intermediate in the synthesis of compound 6 described above) (330 mg, 0.70 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 700 mg). The crude product was purified and separated using a Flash column (4 g silica gel; the mobile phases were respectively: 100 mL of *n*-heptane; 100 mL of 1% EtOAc + 99% *n-*heptane; 100 mL of 2% EtOAc + 98% *n*-heptane; 100 mL of 8% EtOAc + 92% *n*-heptane; 100 mL of 20% EtOAc + 300 mL of 80% *n*-heptane + 30% EtOAc + 70% *n*-heptane) to obtain a pure product (compound 9, 140 mg, 27% yield). ¹H NMR (400 MHz, CDCl₃) δ 6.33 (s, 1H), 5.02 (p, *J* = 5.8 Hz, 1H), 4.05 (t, *J* = 6.7 Hz, 4H), 3.57-3.42 (m, 2H), 2.49 (qd, *J* = 12.9, 6.1 Hz, 2H), 2.38-2.24 (m, 11H), 2.17 (t, *J* = 7.7 Hz, 2H), 1.72-1.51 (m, 12H), 1.50-1.16 (m, 43H), 0.96-0.78 (m, 9H).

Compound 10 was synthesized according to the following reaction scheme:

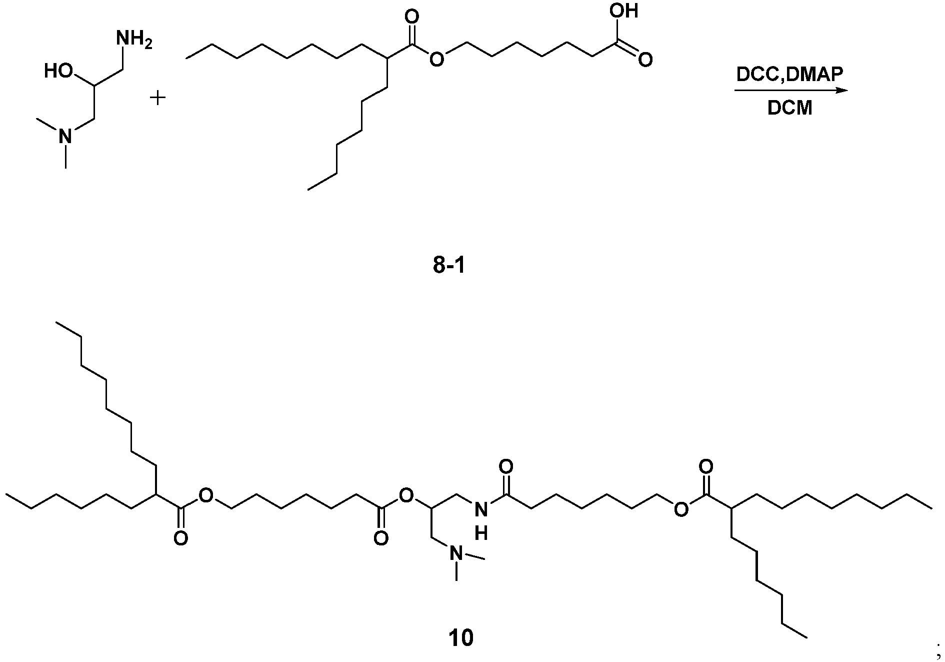

### Step 1: synthesis of compound 10

6-Carboxyhexyl 2-hexyldecanoate (compound 8-1, CAS: 2805989-46-0) (500 mg, 1.3 mmol), 1,3-dicyclohexylcarbodiimide DCC (268.24 mg, 1.3 mmol), 4-dimethylaminopyridine DMAP (39.71 mg, 0.33 mmol), and dichloromethane DCM (3 mL) were added into a 25 mL flask, and the mixture was stirred at room temperature for 30 min. 1-Amino-3-(dimethylamino)propan-2-ol (76.82 mg, 0.65 mmol) was then added dropwise, and the mixture was stirred at room temperature overnight. The reaction progress was monitored by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to obtain a crude product (about 700 mg). The crude product was purified and separated using a Flash column (4 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane + 200 mL of 30% EtOAc + 70% *n*-heptane) to obtain a pure product (compound 10, 120 mg, 22% yield). ¹H NMR (400 MHz, CDCl₃) δ 6.40 (s, 1H), 5.04 (q, *J* = 6.0 Hz, 1H), 4.05 (td, *J* = 6.7, 2.6 Hz, 4H), 3.50 (t, *J* = 5.6 Hz, 2H), 2.68-2.46 (m, 2H), 2.31 (d, *J* = 12.8, 3.8 Hz, 10H), 2.17 (t, *J* = 7.6 Hz, 2H), 1.60 (dt, *J* = 22.6, 7.8 Hz, 13H), 1.50-1.15 (m, 61H), 0.87 (t,*J* = 6.7 Hz, 12H).

### Example 2: Preparation of Lipid Nanoparticle TMF

In this example, TMF1 to TMF17 refer to lipid nanoparticles formed using the compounds prepared in Example 1 as cationic lipids and other components, with no limitation on the mRNA encapsulated therein.

Preparation of lipid nanoparticle TMF1 loaded with luciferase mRNA (hereinafter referred to as Luc-mRNA) (containing 46.3% compound 1):
1) Compound 1 (cationic lipid), DOPE (helper lipid), cholesterol (helper lipid), and PEG2000-DMG (helper lipid) were dissolved with ethanol to obtain oil phase (ethanol phase) stock solutions at molar percentages of 46.3%, 9.4%, 42.7%, and 1.6%, respectively;
2) The Luc-mRNA stock was diluted to 0.3 mg/mL with pH 4 citrate buffer to obtain an aqueous phase;
3) The oil phase (ethanol phase) containing the mixture of four lipids obtained in step 1) and the aqueous phase containing mRNA obtained in step 2) were rapidly mixed in a volume ratio of 1:3 to prepare TMF1 with a weight ratio of total lipids to mRNA of 24.5:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Lipid nanoparticles TMF2 to TMF17 were prepared with reference to the preparation method for TMF1, wherein the lipid composition was replaced according to the lipids listed in Table 1.

**Table 1. Lipid composition of lipid nanoparticles TMF1 to TMF17**

| Lipid nanoparticle No. | Cationic lipid | Helper lipid 1 | Helper lipid 2 | Helper lipid 3 | Number of component types |
|---|---|---|---|---|---|
| TMF1 | 46.3% compound 1 | 9.4% DOPE | 42.7% cholesterol | 1.6% PEG2000-DMG | Four |
| TMF2 | 46.3% compound 2 | 9.4% DOPE | 42.7% cholesterol | 1.6% PEG2000-DMG | Four |
| TMF3 | 46.3% compound 3 | 9.4% DOPE | 42.7% cholesterol | 1.6% PEG2000-DMG | Four |
| TMF4 | 46.3% compound 4 | 9.4% DOPE | 42.7% cholesterol | 1.6% PEG2000-DMG | Four |
| TMF5 | 46.3% compound 5 | 9.4% DOPE | 42.7% cholesterol | 1.6% PEG2000-DMG | Four |
| TMF6 | 46.3% compound 6 | 9.4% DOPE | 42.7% cholesterol | 1.6% PEG2000-DMG | Four |
| TMF7 | 46.3% compound 7 | 9.4% DOPE | 42.7% cholesterol | 1.6% PEG2000-DMG | Four |
| TMF8 | 46.3% compound 8 | 9.4% DOPE | 42.7% cholesterol | 1.6% PEG2000-DMG | Four |
| TMF9 | 46.3% compound 9 | 9.4% DOPE | 42.7% cholesterol | 1.6% PEG2000-DMG | Four |
| TMF10 | 46.3% compound 10 | 9.4% DOPE | 42.7% cholesterol | 1.6% PEG2000-DMG | Four |
| TMF11 | 46.3% compound 5 | 9.4% DSPC | 42.7% cholesterol | 1.6% PEG2000-DMG | Four |
| TMF12 | 46.3% compound 7 | 9.4% DSPC | 42.7% cholesterol | 1.6% PEG2000-DMG | Four |
| TMF13 | 46.3% compound 10 | 9.4% DSPC | 42.7% cholesterol | 1.6% PEG2000-DMG | Four |
| TMF14 | 46.3% compound 004 | 9.4% DSPC | 42.7% cholesterol | 1.6% PEG2000-DMG | Four |
| TMF15 | 46.3% compound 004 | 9.4% DOPE | 42.7% cholesterol | 1.6% PEG2000-DMG | Four |
| TMF16 | 46.3% compound DODAP | 9.4% DOPE | 42.7% cholesterol | 1.6% PEG2000-DMG | Four |
| TMF17 | 46.3% compound ALC-0315 | 9.4% DOPE | 42.7% cholesterol | 1.6% PEG2000-DMG | Four |

In addition, ALC-0315, DODAP, and 004 were used as cationic lipid controls, and the structures are shown below:

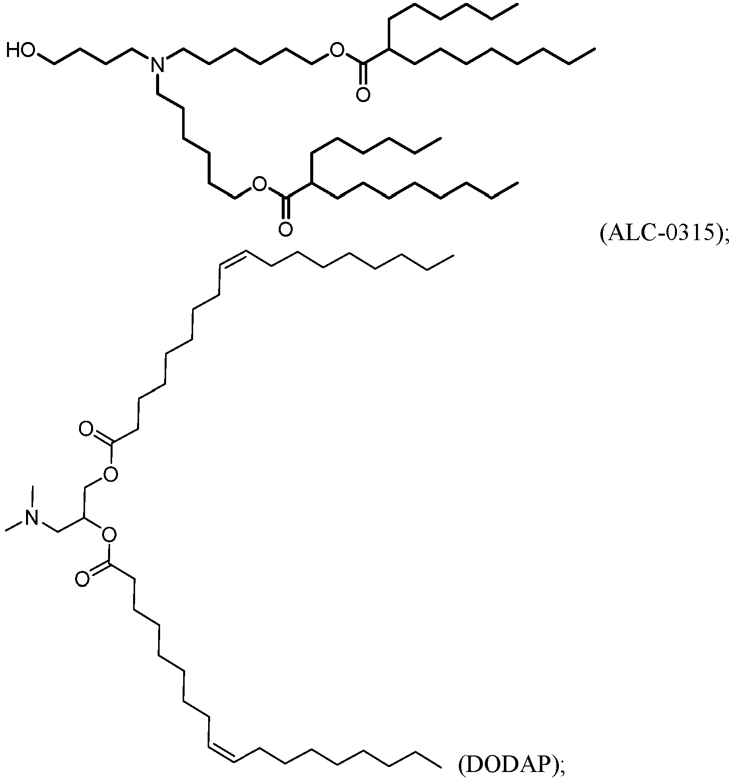

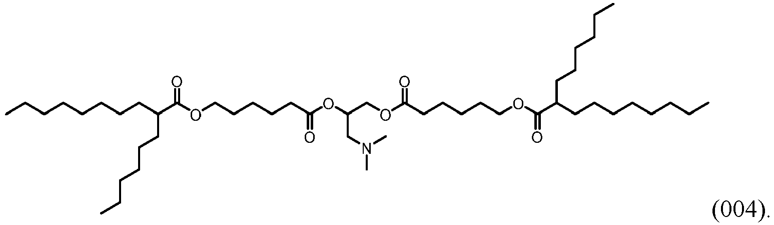

### Example 3: Characterization Detection of Lipid Nanoparticles

The particle size and polydispersity index (PDI) of the lipid nanoparticles were measured using NanoBrook 90plus PLAS (Brookhaven Instruments, US) by dynamic light scattering (DLS) technology at a side scatter angle of 90°. The measurement results are shown in Table 2 below.

**Table 2: Characterization data for lipid nanoparticles prepared from representative cationic lipid compounds**

| Lipid nanoparticle No. | Cationic lipid | Helper lipid | Number of component types | Particle size (nm) | PDI (polydispersity index) |
|---|---|---|---|---|---|
| TMF1 | 46.3% compound 1 | 9.4% DOPE | Four | 92.4±0.47 | 0.068±0.022 |
| TMF2 | 46.3% compound 2 | 9.4% DOPE | Four | 113.5±0.63 | 0.078±0.032 |
| TMF3 | 46.3% compound 3 | 9.4% DOPE | Four | 121.5±1.02 | 0.085±0.024 |
| TMF4 | 46.3% compound 4 | 9.4% DOPE | Four | 98.7±0.58 | 0.103±0.010 |
| TMF5 | 46.3% compound 5 | 9.4% DOPE | Four | 101.1±0.47 | 0.068±0.015 |
| TMF6 | 46.3% compound 6 | 9.4% DOPE | Four | 113.3±0.76 | 0.086±0.018 |
| TMF7 | 46.3% compound 7 | 9.4% DOPE | Four | 170.6±2.45 | 0.153±0.004 |
| TMF8 | 46.3% compound 8 | 9.4% DOPE | Four | 106.0±0.64 | 0.088±0.021 |
| TMF9 | 46.3% compound 9 | 9.4% DOPE | Four | 130.4±0.55 | 0.053±0.006 |
| TMF10 | 46.3% compound 10 | 9.4% DOPE | Four | 103.4±1.04 | 0.077±0.008 |
| TMF11 | 46.3% compound 5 | 9.4% DSPC | Four | 106.0±1.10 | 0.124±0.022 |
| TMF12 | 46.3% compound 7 | 9.4% DSPC | Four | 153.5±0.04 | 0.105±0.01 |
| TMF13 | 46.3% compound 10 | 9.4% DSPC | Four | 99.9±0.63 | 0.13±0.004 |
| TMF14 | 46.3% compound 004 | 9.4% DSPC | Four | 112.1±0.87 | 0.106±0.042 |
| TMF15 | 46.3% compound 004 | 9.4% DOPE | Four | 103.6±0.61 | 0.039±0.012 |
| TMF16 | 46.3% compound DODAP | 9.4% DOPE | Four | 103.7±0.43 | 0.062±0.013 |
| TMF17 | 46.3% compound ALC-0315 | 9.4% DOPE | Four | 106.4±0.75 | 0.049±0.003 |

### Example 4: Delivery System In Vivo Distribution 210

The lipid nanoparticle loaded with Luc-mRNA prepared in Example 2 was administered by both intravenous and intramuscular injections.

Intravenous injection: The liposomes prepared in Example 2 were injected at a dose of 0.5 mg/kg through the inner canthus venous plexus of the fundus to 6-8 week-old female ICR mice. 4 h after the administration, 15 mg/mL D-luciferin potassium salt was intraperitoneally injected at a dose of 150 mg/kg. 10 min after injection of the luciferase substrate, mice were placed under an *in vivo* imaging system (IVIS Lumina XRMS Series III, PerkinElmer) and observed for *in vivo* fluorescence intensity and distribution. The mice were then sacrificed, organs (heart, liver, spleen, lung and kidney) were isolated and imaged *ex vivo* to observe the fluorescence intensity and distribution in the different organs.

The distribution of Luc-mRNA delivered by representative lipid compounds in the organs is shown in Table 3. As shown in Table 3, the fluorescence intensity of the lipid nanoparticle TMF5 in the spleen was higher than that of the control groups TMF15, TMF16, and TMF17. Moreover, the ratio of fluorescence intensity in the spleen to that in the liver (spleen/liver) was higher than that of the control groups TMF16 and TMF17 and lower than that ofTMF15.

Intramuscular injection: The liposomes prepared in Example 2 were injected at a dose of 0.5 mg/kg through the inner thigh muscle to 6-8 week-old female ICR mice. 4 h after the administration, 15 mg/mL D-luciferin potassium salt was intraperitoneally injected at a dose of 150 mg/kg. 10 min after injection of the luciferase substrate, mice were placed under an *in vivo* imaging system (IVIS Lumina XRMS Series III, PerkinElmer) and observed for *in vivo* fluorescence intensity and distribution.

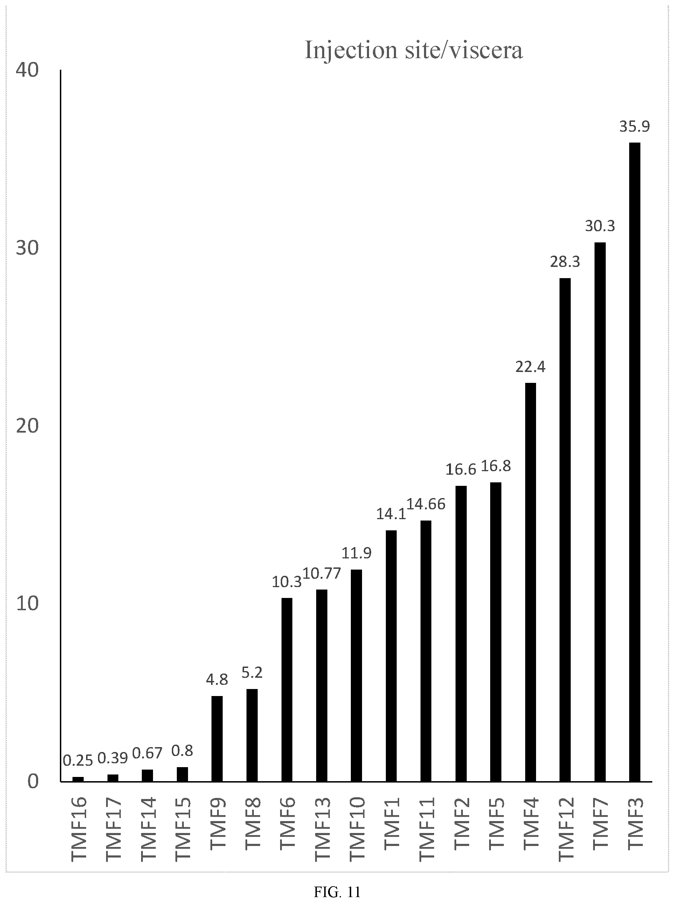

The *in vivo* distribution of Luc-mRNA delivered by representative lipid compounds is shown in Table 4. In addition, FIG. 11 shows a summary of the fluorescence intensity ratios of these lipid nanoparticles at the injection site and viscera.

As shown in Table 4 and FIG. 11, the ratio of fluorescence intensity at the injection site to that in the viscera reached 30 or more for both the lipid nanoparticles TMF7 and TMF3 containing compound 7 and compound 3, respectively, 4 hours after administration by intramuscular injection in mice, wherein the ratio for TMF3 was the highest, reaching 35.9. The fluorescence intensity of the lipid nanoparticles TMF7, TMF10, TMF12, and TMF13 at the injection site was relatively high, which were all 6.5E+09 or more, wherein the intensity of TMF7 was the highest, reaching 8.52E+09. Comparing TMF5 and TMF11, TMF7 and TMF12, and TMF10 and TMF13, the data show that lipid nanoparticles prepared with the same cationic lipid and different phospholipids have similar fluorescence intensities at the injection site and similar ratios of fluorescence intensities at the injection site to that in the viscera.

Compared with the control lipid nanoparticles TMF14 and TMF15 using compound 004, the lipid nanoparticles TMF1 to TMF13 of the present disclosure not only had higher selectivity at the injection site than that of the control group, but also had higher fluorescence intensity at the injection site than that of the control group by 3 orders of magnitude or more; compared with the control lipid nanoparticle TMF16 using DODAP, the lipid nanoparticle TMF1 to TMF13 of

the present application not only had higher selectivity at the injection site than that of the control group, but also had higher fluorescence intensity at the injection site than that of the control group by 3 orders of magnitude or more; compared with the control lipid nanoparticle TMF17 using ALC-0315, the lipid nanoparticle TMF1 to TMF13 of the present application not only had higher selectivity at the injection site than that of the control group, but also had higher fluorescence intensity at the injection site than that of the control group by 1 order of magnitude or more.

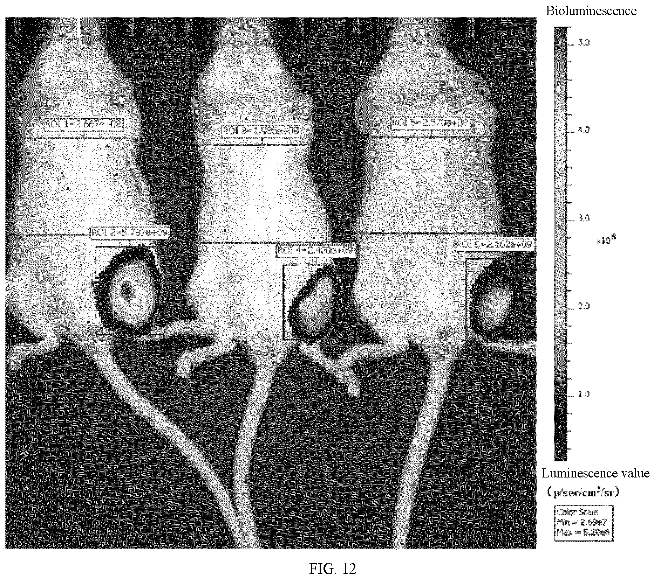

In addition, FIG. 12 also shows the fluorescence signal of mice *in vivo* 4 h after administration by intramuscular injection of the lipid nanoparticle TMF1 loaded with Luc-mRNA; as shown in FIG. 12, the fluorescence signal was strongest at the injection site of the mice, and very weak in the viscera. This further shows that the expression of Luc-mRNA loaded on TMF1 at the injection site was significantly higher than that in the viscera.

The fluorescence intensity in different sites represents the delivery efficiency of the corresponding delivery system at different sites. The above data fully demonstrate that the lipid nanoparticles of the present application are able to successfully deliver and highly express nucleic acid molecules into injection sites, and the delivery efficiency for injection sites is significantly higher than that for viscera, reducing potential hepatotoxicity.

**Table 3: Expression intensity of Luc-mRNA delivered by lipid nanoparticles prepared from representative cationic lipids (IV)**

| Lipid nanoparticle No. | Fluorescence intensity | | |
|---|---|---|---|
| | Spleen [p/s] | Liver [p/s] | Spleen/liver |
| TMF5 | 3.19E+08 | 1.59E+08 | 2.5 |
| TMF15 | 1.19E+08 | 1.52E+06 | 60.6 |
| TMF16 | 1.45E+06 | 1.69E+06 | 0.9 |
| TMF17 | 3.52E+07 | 1.99E+09 | 0.02 |

**Table 4: Expression intensity of Luc-mRNA delivered by lipid nanoparticles prepared from representative cationic lipids (IM)**

| Lipid nanoparticle No. | Fluorescence intensity | | |
|---|---|---|---|
| | Injection site [p/s] | Viscera [p/s] | Injection site/viscera |
| TMF1 | 3.46E+09 | 2.41E+08 | 14.10 |
| TMF2 | 1.61E+09 | 9.70E+07 | 16.60 |
| TMF3 | 1.18E+09 | 3.29E+07 | 35.90 |
| TMF4 | 4.42E+09 | 1.97E+08 | 22.40 |
| TMF5 | 5.74E+09 | 3.42E+08 | 16.80 |
| TMF6 | 4.28E+09 | 6.10E+08 | 10.30 |
| TMF7 | 8.52E+09 | 2.81E+08 | 30.30 |
| TMF8 | 4.18E+09 | 1.19E+09 | 5.20 |
| TMF9 | 7.32E+08 | 1.53E+08 | 4.80 |
| TMF10 | 6.53E+09 | 5.49E+08 | 11.90 |
| TMF11 | 5.44E+09 | 3.71E+08 | 14.66 |
| TMF12 | 7.52E+09 | 2.66E+08 | 28.30 |
| TMF13 | 6.23E+09 | 5.78E+08 | 10.77 |
| TMF14 | 5.63E+06 | 8.40E+06 | 0.67 |
| TMF15 | 5.63E+06 | 7.04E+06 | 0.80 |
| TMF16 | 3.69E+05 | 1.45E+06 | 0.25 |
| TMF17 | 1.41E+08 | 3.86E+08 | 0.39 |

The present disclosure is described above in detail so that those skilled in the art can understand the mechanisms and content of the present disclosure and implement it. However, the description does not limit the protection scope of the present disclosure, and any equivalent changes or modifications made in line with the spirit of the present disclosure shall fall within the protection scope of the present disclosure.

## Claims

1. A compound represented by formula (I) 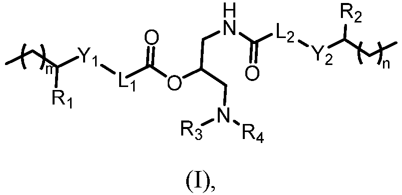
or a pharmaceutically acceptable salt, a prodrug, or a stereoisomer thereof,
wherein:
Y₁ and Y₂ are each independently -OC (=O)- or -C (=O)O-;
L₁ and L₂ are each independently a bond or optionally substituted C₁-C₁₈ alkylene;
R₁ and R₂ are each independently H or C₁-C₈ linear alkyl or C₁-C₈ branched alkyl;
R₃ and R₄ are each independently C₁-C₈ linear alkyl or C₁-C₈ branched alkyl, optionally substituted with hydroxyl;
m is an integer of 1-10; and
n is an integer of 1-10.

2. The compound or the pharmaceutically acceptable salt, the prodrug, or the stereoisomer thereof according to claim 1, wherein L₁ and L₂ are each independently unsubstituted C₂-C₁₄ alkylene; and/or R₁ and R₂ are each independently H or C₁-C₈ linear alkyl;
and/or R₃ and R₄ are each independently C₁-C₄ linear alkyl or C₁-C₈ branched alkyl, optionally substituted with hydroxyl;
and/or m is an integer of 5-7;
and/or n is an integer of 5-7.

3. The compound or the pharmaceutically acceptable salt, the prodrug, or the stereoisomer thereof according to claim 2, wherein R₁ and R₂ are each independently H or C₆-C₈ linear alkyl;
and/or R₃ and R₄ are each independently methyl, ethyl, propyl, hydroxymethyl, hydroxyethyl, hydroxypropyl, or hydroxybutyl.

4. The compound or the pharmaceutically acceptable salt, the prodrug, or the stereoisomer thereof according to claim 1, wherein the compound is selected from the following compounds: 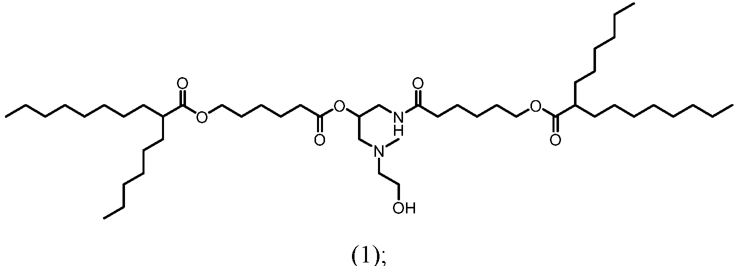 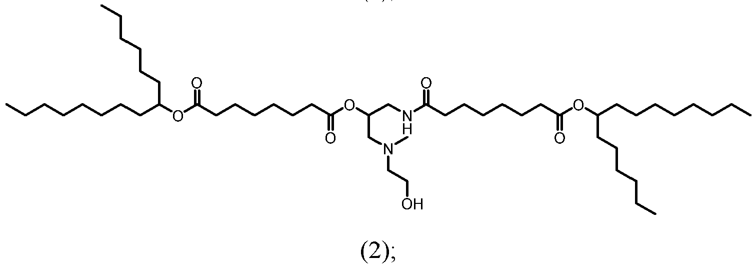 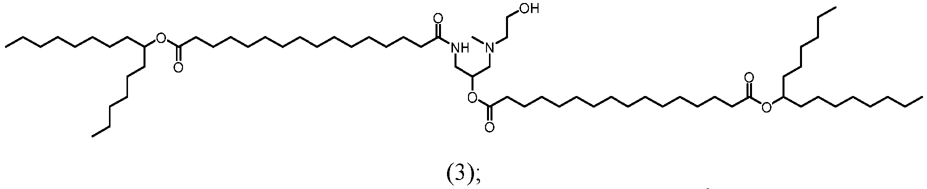 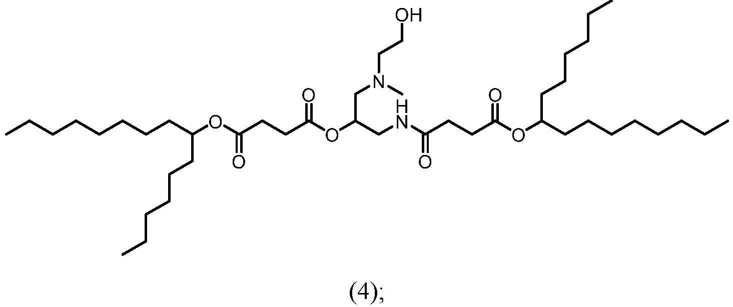 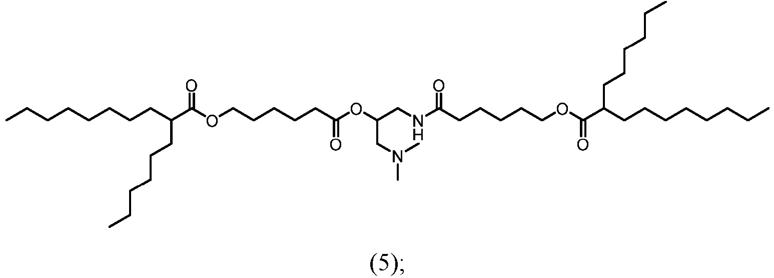 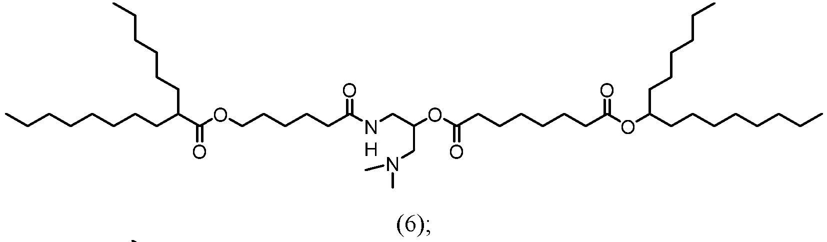 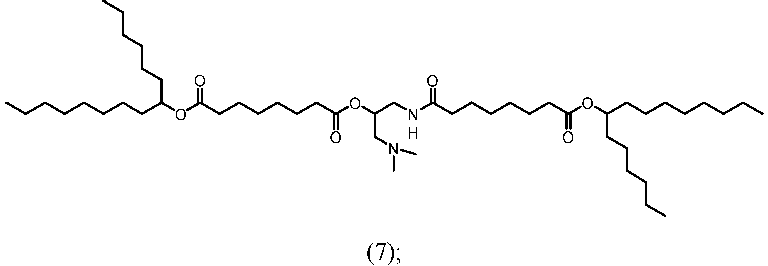 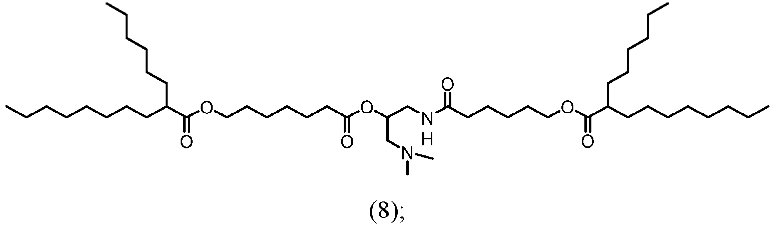 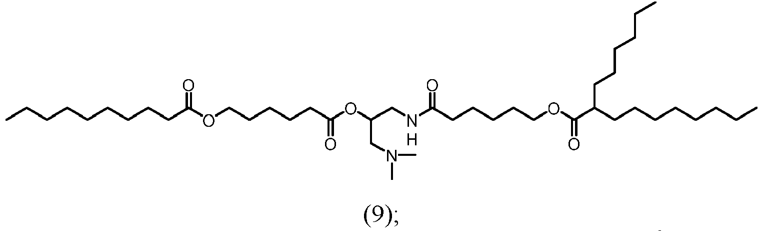 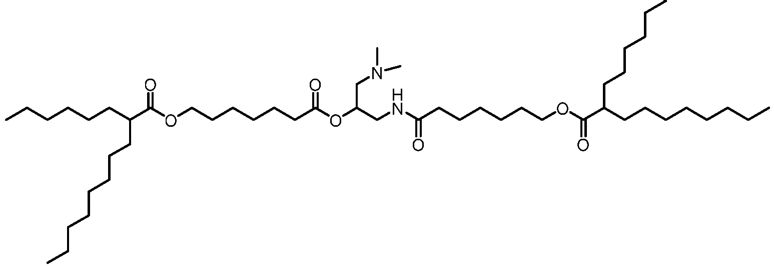 (10), or a pharmaceutically acceptable salt, a prodrug, or a stereoisomer thereof.

5. A lipid nanoparticle, comprising the compound or the pharmaceutically acceptable salt, the prodrug, or the stereoisomer thereof according to any one of claims 1-4.

6. The lipid nanoparticle according to claim 5, wherein the lipid nanoparticle further comprises a helper lipid selected from one or more of a phospholipid, a steroid, a polymer conjugated lipid, and a modifiable lipid.

7. The lipid nanoparticle according to claim 6, wherein the compound or the pharmaceutically acceptable salt, the prodrug, or the stereoisomer thereof and the helper lipid are in a molar ratio of 1:(0.5-2).

8. The compound or the pharmaceutically acceptable salt, the prodrug, or the stereoisomer thereof according to any one of claims 1-4, or the lipid nanoparticle according to any one of claims 5-7 for use in preparing a drug delivery carrier.

9. A pharmaceutical composition comprising the lipid nanoparticle according to any one of claims 5-8 and a therapeutic agent or prophylactic agent.

10. The pharmaceutical composition according to claim 9, wherein the therapeutic agent or prophylactic agent is a nucleic acid, and the nucleic acid is encapsulated in the lipid nanoparticle.

11. The pharmaceutical composition according to claim 10, wherein the nucleic acid is selected from a single-stranded DNA, a double-stranded DNA, a single-stranded RNA, a double-stranded RNA, a short isomer, a plasmid DNA, a complementary DNA, an antisense oligonucleotide, a small interfering nucleic acid, a small activating nucleic acid, an asymmetric interfering nucleic acid, a micro nucleic acid, an agomir, an antagomir, a Dicer enzyme substrate nucleic acid, a small hairpin nucleic acid, a transfer RNA, a messenger RNA, a circular RNA, a self-amplifying mRNA, and an aptamer.

12. The pharmaceutical composition according to claim 9, wherein, in the pharmaceutical composition, the lipid nanoparticle and the therapeutic agent or prophylactic agent are in a mass ratio of 10:1 to 100:1.

13. The pharmaceutical composition according to claim 9, wherein the pharmaceutical composition has an average particle size of 90 nm to 600 nm;
and/or the pharmaceutical composition has a polydispersity index of 0.001 to 0.5.

14. The pharmaceutical composition according to any one of claims 9-13 for use in preparing a targeted delivery drug.

15. A formulation comprising the pharmaceutical composition according to any one of claims 9-14 and a pharmaceutically acceptable auxiliary material.
